# EUROPEAN PATENT APPLICATION

(11) **EP 1 400 593 A1**
(43) Date of publication of application: **24.03.2004**
(21) Application number: 02733320.2
(22) Date of filing: 05.06.2002
(51) Int. Cl.: C12N 15/67, C12N 1/21, C12P 21/00

(54) **NOVEL PROMOTER**

(30) Priority: 06.06.2001 JP 2001171607
(71) Applicant: Takeda Chemical Industries, Ltd., Osaka-shi, Osaka 541-0045 (JP)
(72) Inventor: ITO, Takashi, Tsukuba-shi, Ibaraki 305-0821 (JP); TANAKA, Yoko, Kyotanabe-shi, Kyoto 610-0331 (JP); KURIYAMA, Masato, Osaka-shi, Osaka 534-0016 (JP)
(74) Representative: Rickard, Timothy Mark Adrian
(86) International application number: PCT/JP2002/005555
(87) International publication number: WO 2002/101058

(57) **Abstract**

The present invention relates a promoter capable of strongly expressing a gene. linked downstream (3' side) of the promoter in prokaryote, especially in *Escherichia Coli*. The promoter of the invention is a promoter for prokaryote having a novel nucleotide sequence between any -35 region sequence and any -10 region sequence. The promoter has a strong promoter activity and thus a target peptide or protein can be produced at a high efficiency and in large amounts through linking the structural gene encoding the target peptide or protein downstream of the promoter.

Sequence Listing

<110> Takeda Chemical Industries, Ltd.

<120> Novel Promoter

<130> P02-0062PCT

<140> PCT/JP02/05555
<141> 2002-06-05

<150> JP 2001-171607
<151> 2001-06-06

<160> 39

<210> 1
<211> 28
<212> DNA
<213> Artificial sequence

<220>
<223> Primer

<400> 1
cgttatccgg atcacatgaa acggcatg     28

<210> 2
<211> 28
<212> DNA
<213> Artificial sequence

<220>
<223> Primer

<400> 2

catgccgttt catgtgatcc ggataacg     28

<210> 3
<211> 33
<212> DNA
<213> Artificial sequence

<220>
<223> Synthetic DNA

<400> 3
agcttcatat gttcgaagta ctagatctgg tac     33

<210> 4
<211> 25
<212> DNA
<213> Artificial sequence

<220>
<223> Synthetic DNA

<400> 4
cagatctagt acttcgaaca tatga     25

<210> 5
<211> 109
<212> DNA
<213> Artificial sequence

<220>
<223> Synthetic DNA

<400> 5
aattctataa aaataattgt tgacatattt tataaatttt ggcataatag atctaattgt 60
gagcggataa caattctgca gaagcttgag ctcggtaccc ggggatcct     109

<210> 6
<211> 109
<212> DNA
<213> Artificial sequence

<220>
<223> Synthetic DNA

<400> 6
ctagaggatc cccgggtacc gagctcaagc ttctgcagaa ttgttatccg ctcacaatta 60
gatctattat gccaaaattt ataaaatatg tcaacaatta tttttatag 109

<210> 7
<211> 69
<212> DNA
<213> Artificial sequence

<220>
<223> Synthetic DNA

<400> 7
tgagcatgca tactagtctc gagtaatccc acagccgccg ccagttccgc tggcggcggc 60
attttcgat 69

<210> 8
<211> 64
<212> DNA
<213> Artificial sequence

<220>
<223> Synthetic DNA

<400> 8
cgaaaatgcc gccgccagcg gaactggcgg cggctgtggg attactcgag actagtatgc 60
atgc 64

<210> 9
<211> 63
<212> DNA
<213> Artificial sequence

<220>
<223> Synthetic DNA

<400> 9
ctagcgaatt cgagcatatg agcactagtg catgcgagcc atattcaacg ggaaacgtct 60
tgc 63

<210> 10
<211> 63
<212> DNA
<213> Artificial sequence

<220>
<223> Synthetic DNA

<400> 10
tcgagcaaga cgtttcccgt tgaatatggc tcgcatgcac tagtgctcat atgctcgaat 60
tcg 63

<210> 11
<211> 24
<212> DNA
<213> Artificial sequence

<220>
<223> Synthetic DNA

<400> 11
tatgagggta ccgccggctg catg 24

<210> 12

<211> 18
<212> DNA
<213> Artificial sequence

<220>
<223> Synthetic DNA

<400> 12
cagccggcgg taccctca                    18

<210> 13
<211> 36
<212> DNA
<213> Artificial sequence

<220>
<223> Synthetic DNA

<400> 13
gatgagctct acaaataata aattccaact gagcgc          36

<210> 14
<211> 36
<212> DNA
<213> Artificial sequence

<220>
<223> Synthetic DNA

<400> 14
gcgctcagtt ggaatttatt atttgtagag ctcatc          36

<210> 15
<211> 73
<212> DNA
<213> Artificial sequence

<220>
<221> variation
<222> (19)...(35)
<223> Synthetic DNA; n is a, c, g or t.

<400> 15
acaattagat ctattatgnn nnnnnnnnnn nnnnntgtca acaattattt ttatagaatt 60
catcgataag ctt 73

<210> 16
<211> 18
<212> DNA
<213> Artificial sequence

<220>
<223> Synthetic DNA

<400> 16
aagcttatcg atgaattc 18

<210> 17
<211> 27
<212> DNA
<213> Artificial sequence

<220>
<223> Synthetic DNA

<400> 17
ttcatacacg gtgcctgact gcgttag 27

<210> 18
<211> 23
<212> DNA
<213> Artificial sequence

<220>
<223> Synthetic DNA

<400> 18
tcaacaagaa ttgggacaac tcc                23

<210> 19
<211> 48
<212> DNA
<213> Artificial sequence

<220>
<223> Synthetic DNA

<400> 19
aattctataa aaataattgt tgacaattaa tcatcggctc gtataata          48

<210> 20
<211> 48
<212> DNA
<213> Artificial sequence

<220>
<223> Synthetic DNA

<400> 20
gatctattat acgagccgat gattaattgt caacaattat ttttatag          48

<210> 21
<211> 49
<212> DNA
<213> Artificial sequence

<220>
<223> Synthetic DNA

<400> 21
aattctataa aaataattgt tgacaacgcg cccagcgagt actataata          49

<210> 22
<211> 49
<212> DNA
<213> Artificial sequence

<220>
<223> Synthetic DNA

<400> 22
gatattttta ttaacaactg ttgcgcgggt cgctcatgat attatctag          49

<210> 23
<211> 46
<212> DNA
<213> Artificial sequence

<220>
<223> Synthetic DNA

<400> 23
tatgttccca accattccct tatccaggct ttttgacaac gcttta          46

<210> 24
<211> 48
<212> DNA
<213> Artificial sequence

<220>
<223> Synthetic DNA

<400> 24
ccggtaaagc gttgtcaaaa agcctggata agggaatggt tgggaaca          48

<210> 25
<211> 75
<212> DNA
<213> Artificial sequence

<220>
<221> variation
<222> (37), (40), (43), (49), (52)
<223> Synthetic DNA; n is a, c, g or t.

<400> 25
tcaagagttt accggtaaag cgttgtcaaa aagcctngan agnggdatng tnggraacat 60
atgttggcgc gcctt 75

<210> 26
<211> 75
<212> DNA
<213> Artificial sequence

<220>
<221> variation
<222> (40), (43), (49), (52)
<223> Synthetic DNA; n is a, c, g or t.

<400> 26
tcaagagttt accggtaaag cgttgtcaaa aagcctrctn agnggdatng tnggraacat 60
atgttggcgc gcctt 75

<210> 27
<211> 75
<212> DNA
<213> Artificial sequence

<220>
<221> variation

<222> (37), (43), (49), (52)
<223> Synthetic DNA; n is a, c, g or t.

<400> 27
tcaagagttt accggtaaag cgttgtcaaa aagcctngay aanggdatng tnggraacat 60
atgttggcgc gcctt 75

<210> 28
<211> 75
<212> DNA
<213> Artificial sequence

<220>
<221> variation
<222> (43), (49), (52)
<223> Synthetic DNA; n is a, c, g or t.

<400> 28
tcaagagttt accggtaaag cgttgtcaaa aagcctrcty aanggdatng tnggraacat 60
atgttggcgc gcctt 75

<210> 29
<211> 18
<212> DNA
<213> Artificial sequence

<220>
<223> Synthetic DNA

<400> 29
aaggcgcgcc aacatatg 18

<210> 30
<211> 30
<212> DNA
<213> Artificial sequence

<220>
<223> Synthetic DNA

<400> 30
atatgctaag agcccatcgt ctgcaccagc          30

<210> 31
<211> 24
<212> DNA
<213> Artificial sequence

<220>
<223> Synthetic DNA

<400> 31
ctgtaggtct gcttgaagat ctgc          24

<210> 32
<211> 35
<212> DNA
<213> Artificial sequence

<220>
<223> Synthetic DNA

<400> 32
tatgtttccc accataccct tatcaaggct ttttg          35

<210> 33
<211> 33
<212> DNA
<213> Artificial sequence

<220>
<223> Synthetic DNA

<400> 33
caaaaagcct tgataagggt atggtgggaa aca          33

<210> 34
<211> 17
<212> DNA
<213> Artificial sequence

<220>
<223> novel promoter sequence

<400> 34
acgcgcccag cgagtac          17

<210> 35
<211> 29
<212> DNA
<213> Artificial sequence

<220>
<223> NP3 promotor

<400> 35
ttgacaacgc gcccagcgag taccataat     29

<210> 36
<211> 29
<212> DNA
<213> Artificial sequence

<220>
<223> trp3 promotor

<400> 36
ttgacaacgc gcccagcgag tactatact     29

<210> 37
<211> 6
<212> DNA
<213> E.coli

<220>
<223> -35 region

<400> 37
ttgaca                                    6

<210> 38
<211> 6
<212> DNA
<213> E.coli

<220>
<223> -10 region

<400> 38
cataat                                    6

<210> 39
<211> 6
<212> DNA
<213> E.coli

<220>
<223> -10 region

<400> 39
tatact                                    6

## Description

### TECHNICAL FIELD

The present invention relates a promoter capable of strongly expressing a gene linked downstream (3' side) of the promoter in prokaryote, especially *Escherichia Coli.*

### BACKGROUND ART

When a protein is expressed in a prokaryotic or eukaryotic host cell using the recombinant DNA technology, the protein expression efficiency is greatly influenced particularly by the efficiency of mRNA transcription of the structural gene encoding the protein.

The transcription efficiency refers to an efficiency at which RNA polymerase starts mRNA synthesis, and generally it is highly dependent on the strength of promoter present upstream of the structural gene. In prokaryote, the promoter strength is mainly controlled by -10 region (so-called Pribnow sequence) located around 10 bp upstream from the transcriptional start point, and -35 region located around 35 bp upstream from the transcriptional start point in the promoter nucleotide sequence. The transcription of a gene is the first step for the gene expression (i.e. the protein expression). In order to produce a desired protein or peptide in large quantity using the recombinant DNA technology, it is required to use a strong promoter which enables a more efficient transcription.

To date, there have been known promoters used for the protein expression in *Escherichia coli,* such as tryptophan promoter derived from E. coli (referred to as "trp"; Emtage, J.S. et al., Nature 283, 171, 1983), lactose promoter derived from E. coli (referred to as "lac"; Itakura, K., Science 198, 1056, 1977), and PL promoter of E. coli phage λ (Bernard, H., Gene 5, 59, 1979), but these promoter have a defect of relatively weak protein expression activity.

On the other hand, also provided are hybrid promoters in which a promoter having a relatively strong expression activity is bound to an easily regulatable promoter operator, as described in the patent application publication JP S57-194790. Among them, a hybrid promoter of trp and lac UV5 promoter operator, referred to as "tac", has both strong expression activity and easiness in its regulation, and it is a highly useful promoter operator for the protein production using the genetic recombination (the above-mentioned patent application publication and Proc. Natl. Acad. Sci. USA 80, 21-25, 1983).

However, such a promoter as tac described in the patent application publication JP S57-194790 has a problem that it cannot be easily constructed because two kinds of promoter need to be liked between -35 region and -10 region. In other words, because it is considered that the expression activity of such a promoter is affected by highly conserved nucleotide sequences in -35 and -10 regions, and also by the distance between these regions and the nucleotide sequence therein, it is indispensable to search promoter activities of many candidate sequences prepared as having different nucleotide numbers and sequences between -35 and -10 regions in a case where such a promoter will be constructed.

Further, T7 promoter, especially ϕ10 promoter is known as a promoter having a very strong expression activity (Rosenberg, A.H. et al., Gene 56, 125-135, 1987). However, T7 promoter requires T7 RNA polymerase, and therefore there is a limitation that E. coli lysogenized with λ phage must be used as a host (Studier et al., J. Mol. Biol. 189, 113, 1986).

Accordingly, there is a need to develop a promoter which has a strong expression activity in prokaryote, especially E. coli, and also can be easily constructed.

### DISCLOSURE OF THE INVENTION

The present inventors found a novel sequence between -35 and -10 regions, which is capable of enhancing the expression activity of a promoter for prokaryote. Based on these findings, the inventors successfully developed a novel promoter and achieved the present invention.

The invention provides:
(1 ) A promoter comprising the same or substantially the same nucleotide sequence as that shown by SEQ ID NO: 34.
(2) The promoter according to (1), which comprises the same or substantially the same nucleotide sequence as that shown by SEQ ID NO: 34 between any type of -35 region and any type of -10 region.
(3) The promoter according to (2), in which the -35 region has the nucleotide sequence shown by SEQ ID NO: 37.
(4) The promoter according to (2), in which the -10 region has the nucleotide sequence shown by SEQ ID NO: 38 or 39.
(5) The promoter according to (2), which has the nucleotide sequence shown by SEQ ID NO: 35.
(6) The promoter according to (2), which has the nucleotide sequence shown by SEQ ID NO: 36.
(7) A DNA comprising the promoter according to any one of (1) to (6).
(8) A recombinant vector comprising the promoter according to any one of (1) to (6) or the DNA according to (7).
(9) The recombinant vector according to (8), which comprises a DNA having a structural gene to be expressed under the control of the promoter according to any one of (1) to (6).
(10) The recombinant vector according to (9), referred to as pNP3GHNO12, in which the structural gene is the human gorwth hormone gene.
(11) A transformant transformed with the recombinant vector according to (9).
(12) The transformant *Escherichia coli* MM294/pNP3GHNO12 (FERM BP-7611), which is transformed with the the recombinant vector according to (10).
(13) A method of producing a protein or a salt thereof, which comprises culturing the transformant according to (11) to produce the protein encoded by the structural gene.
(14) A method of producing the human growth hormone or a salt thereof, which comprises culturing the transformant according to (12) to produce the human growth hormone.
(15) The recombinant vector according to (9), in which the structural gene is a reporter gene.
(16) The recombinant vector according to (15), in which the reporter gene is kanamycin resistance gene or GFP gene.
(17) A transformant transformed with the recombinant vector according to (15).
(18) A transformant transformed with the recombinant vector according to (16).
(19) A DNA comprising the same or substantially the same nucleotide sequence as that shown by SEQ ID NO: 34.
(20) The DNA according to (20), which comprises the same or substantially the same nucleotide sequence as that shown by SEQ ID NO: 34 between any type of -35 region and any type of -10 region.
(21) Use of the DNA according to (19) or (20) for the production of a promoter.
(22) Use of the promoter according to any one of (1) to (6) for the production of a protein encoded by a structural gene.
   Further, the invention provides:
(23) A DNA having a promoter region having the same or substantially the same nucleotide sequence as that shown by SEQ ID NO: 34 between any type of -35 region and any type of -10 region.
(24) A recombinant vector comprising the DNA according to (23).
(25) The recombinant vector according to (24), which comprises a DNA having a structural gene to be expressed under the control of a promoter region having the same or substantially the same nucleotide sequence as that shown by SEQ ID NO: 34 between any type of -35 region and any type of -10 region.

### BRIEF DESCRIPTION OF THE DRAWINGS

Fig. 1 shows the construction of plasmid pKMNP. In this figure, Km^{r} indicates kanamycin resistance gene; Amp^{r} indicates ampicilline resistance gene; Tc^{r} indicates tetracycline resistance gene; and Ori indicates the origin of replication.
Fig. 2 shows the construction of plasmid pGFPNPv3.
Fig. 3 shows the construction of plasmid pKMRND.
Fig. 4 shows the construction of plasmid pGFPRND.
Fig. 5 shows the expression efficiency in E. coli clones obtained in Example 4.
Fig. 6 shows the nucleotide sequence of a promoter region derived from E. coli clone No. 6 obtained in Example 4.
Fig. 7 shows the expression efficiency in E. coli clones obtained in Example 5.
Fig. 8 shows the nucleotide sequences of NP3 promoter and trp3 promoter of the invention.
Fig. 9 shows the expression efficiency in E. coli clones obtained in Example 6.
Fig. 10 shows the construction of plasmid pGFPGHNa.
Fig. 11 shows the construction of plasmid pGFPGHNO.
Fig. 12 shows the nucleotide sequence encoding the N-terminal part of the amino acid sequence of human growth hormone, which is contained in E. coli clone No. 12 obtained in Example 7.
Fig. 13 shows the expression efficiency in E. coli clones obtained in Example 7.
Fig. 14 shows the construction of plasmid pTCHGHNO12.
Fig. 15 shows the construction of plasmid pNPHGHNO12.
Fig. 16 shows the construction of plasmid pNP3GHNO12.
Fig. 17 shows the result of cultivation conducted in Example 10.
Fig. 18 shows the result of cultivation conducted in Example 11.

### BEST MODE FOR CARRYING OUT THE INVENTION

The present invention relates to a promoter for prokaryote, which comprises the same or substantially the same nucleotide sequence as that shown by SEQ ID NO: 34 (hereinafter referred to as the promoter of the invention). Particularly preferred is the promoter comprising the same or substantially the same nucleotide sequence as that shown by SEQ ID NO: 34 between any type of -35 region and any type of -10 region. Further preferred is the promoter comprising the nucleotide sequence shown by SEQ ID NO: 34 between any type of -35 region and any type of -10 region.

The said nucleotide sequence that is substantially the same as that shown by SEQ ID NO: 34 includes any nucleotide sequence or DNA sequence, which is hybridizable to the nucleotide sequence shown by SEQ ID NO: 34 under high stringent conditions and has the activity substantially equivalent in property to the promoter activity of the nucleotide sequence shown by SEQ ID NO: 34. The nucleotide sequence placed between -35 region and -10 region has usually 15 to 21 nucleotides, preferably 16 to 18 nucleotides, and particularly 17 nucleotides.

Examples of the DNA that is hybridizable to the nucleotide sequence shown by SEQ ID NO: 34 include a DNA comprising a nucleotide sequence with about 70 % or more, preferably about 80 % or more, more preferably about 90 % or more, the most preferably about 95 % or more homology to the nucleotide sequence shown by SEQ ID NO: 34.

The hybridization can be carried out according to a known method or a modification thereof, for example, the method described in Molecular Cloning, 2nd Ed. (J. Sambrook et al., Cold Spring Harbor Lab. Press, 1989). A commercially available library may also be used according to the method described in the attached manufacturer's instructions. More preferably, the hybridization can be carried out under high stringent conditions.

The high stringent conditions refer to, for example, a sodium concentration of about 19 to 40 mM, preferably about 19 to 20 mM and a temperature of about 50 to 70°C, preferably about 60 to 65°C. In particular, the condition with the sodium concentration of about 19 mM and the temperature of about 65°C is most preferred.

As used herein, the term "-35 region" refers to a sequence region, which is located around 35 bp (base pair) upstream from the transcription start point of genome DNA encoding any protein of prokaryote; which usually consists of 6 to 8 base pairs, preferably 6 base pairs; and which is indispensable for the promoter activity. Examples of -35 region, which may be used, are described in, for example, Nucleic Acid Research vol. 11, no. 8, p. 2238-2242, 1983 and Nucleic Acid Research, vol. 15, no. 5, p. 2243-2361, 1987.

More specifically, nucleotide sequences described in Table 1 can be used as -35 region. Among them, the nucleotide sequence shown by SEQ ID NO: 37 is preferably used.

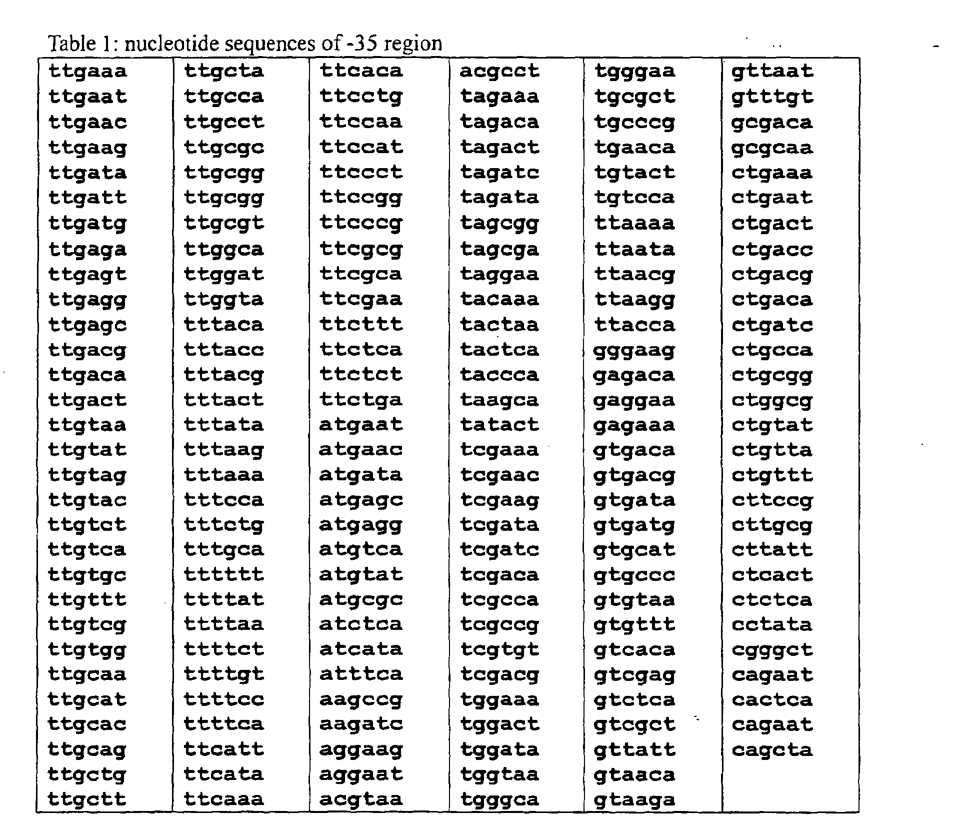

As used herein, the term "-10 region" refers to a sequence region, which is located around 10 bp upstream from the transcription start point of genome DNA encoding any protein of prokaryote; which usually consists of 6 to 8 base pairs, preferably 6 base pairs; and which is indispensable for the promoter activity. Examples of -10 region, which may be used, are described in, for example, Nucleic Acid Research vol. 11, no. 8, p. 2238-2242, 1983 and Nucleic Acid Research, vol. 15, no. 5, p. 2243-2361, 1987.

More specifically, nucleotide sequences described in Table 2 can be used as -10 region. Among them, the nucleotide sequence shown by SEQ ID NO: 38 or NO: 39 is preferably used.

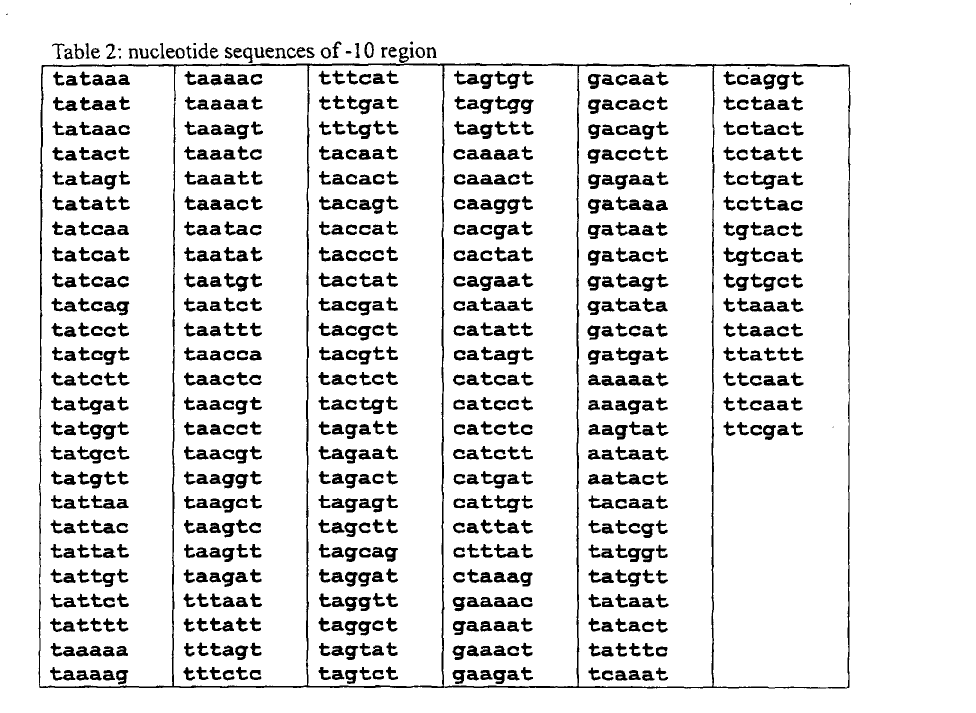

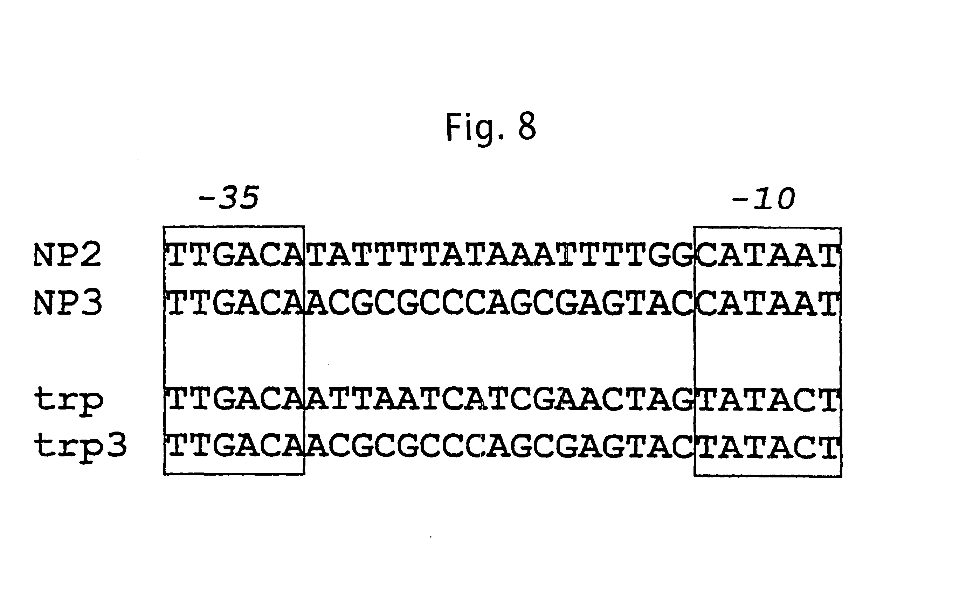

Accrodingly, examples of the promoter of the invention include:
(1) NP3 promoter, which comprises the nucleotide sequence shown by SEQ ID NO: 35 (which comprises the nucleotide sequence shown by SEQ ID NO: 34 between nucleotide sequences shown by SEQ ID NO: 37 and 38)(Fig. 8);
(2) trp3 promoter, which comprises the nucleotide sequence shown by SEQ ID NO: 36 (which comprises the nucleotide sequence shown by SEQ ID NO: 34 between nucleotide sequences shown by SEQ ID NO: 37 and 39)(Fig. 8).

A DNA comprising the same or substantially the same nucleotide sequence as that shown by SEQ ID NO: 34, or a DNA comprising the same or substantially the same nucleotide sequence as that shown by SEQ ID NO: 34 between any type of -35 region and any type of -10 region (hereinafter, the DNA of the invention) is useful as a material for the production of the promoter of the invention as described above.

The DNA and the promoter of the invention can be synthesized according to a well-known method. Nucleotide substitution in the DNA sequence can be conducted by the PCR method or other known methods such as the ODA-LA PCR method, the Gapped duplex method or the Kunkel method, or variations thereof using known kits such as Mutan™-super Express Km (Takara Shuzo Co. Ltd.) or Mutan™-K (Takara Shuzo Co. Ltd.).

The thus obtained DNA or promoter can be used depending upon purpose as it is or if desired, after digestion with a restriction enzyme or after addition of a linker thereto.

The promoter of the invention has an excellent promoter activity, and thus can be used for the preparation of an expression vector, in which a structural gene encoding a desired peptide or protein is operably linked downstream of the promoter. Using the expression vector, the target peptide or protein can be produced efficiently in large amounts.

The said structural gene may be any one derived from any kinds of organisms, provided that the expressed protein is not toxic to the host prokaryote, including for the pharmaceutical industry, genes encoding growth factors, e.g. human growth hormone; interleukins, e.g. IL-1; interferons, e.g. interferon-α; chemokines, e.g. PANTES; and physiologically active peptides, e.g. insulin; and for the research and screening purpose, a gene encoding an unknown protein, which is deduced from genome information.

To investigate the activity of the promoter of the invention, a detectable structural gene, so-called reporter gene may be linked downstream of the promoter. A variety of structural genes may be used as the reporter gene linked downstream of the promoter.

Widly used reporter genes include kanamycin resistance gene, GFP (green fluorescent protein) gene (e.g. GFP, GFPuv) (Jikken Igaku, extra number, Experimantal manuals in the Post-Genomic Era, No. 3, GFP and Bioimaging, published by Yodo-sha (2000)), CAT (chloramphenicol acetyltransferase) gene, alkaline phosphatase gene, and β-galactosidase gene. Any other structural gene can be used in a case where there is a method for detecting the gene product.

Accordingly, the present invention relates to the expression vector for a host prokaryote, which comprises the promoter of the invention. To incorporate the structural gene into the vector, the structural gene may be linked downstream of the promoter region in a direction so that the structural gene can be properly transcripted, and in a position so that the promoter can properly work for the structural gene. Such linkage may be performed using a restriction enzyme cutting site, or a ligase reaction even if there is no proper restriction enzyme site. The expression vector of the invention may contain other elements workable in prokaryote, such as an enhancer sequence for enhancing the activity of the promoter, a transcriptional stop site.

The expression vector comprising the promoter of the invention, as prepared above, can be used to transform a suitable host, and the resultant transformant can be used to produce a protein.

For example, Escherichia bacteria, Bacillus bacteria, and the like are used as a host to be transformed with the recombinant vector as described above.

Examples of Escherichia bacteria include Escherichia coli K12 DH1 (Proc. Natl. Acad. Sci. U.S.A. 60, 160, 1968), JM103 (Nucleic Acids Research 9, 309,1981), JM109, JA221 (Journal of Molecular Biology 120, 517, 1978), HB101 (Journal of Molecular Biology 41, 459, 1969), and C600 (Genetics 39, 440, 1954).

Examples of Bacillus bacteria include Bacillus subtilis MI114 (Gene 24, 255, 1983), 207-21 (Journal of Biochemistry 95, 87, 1984).

More specifically, Escherichia bacteria can be transformed, for example, by the method described in Proc. Natl. Acad. Sci. U.S.A. 69, 2110 (1972) or Gene 17, 107 (1982).

Bacillus bacteria can be transformed, for example, by the method described in Molecular & General Genetics 168, 111 (1979).

The transformant can be cultured according to a well-known method. The transformant derived from an Escherichia or Bacillus bacterium host can be appropriately cultured in a liquid medium, which contains materials required for growth of the transformant such as carbon sources, nitrogen sources, and inorganic materials. Examples of the carbon sources include glucose, dextrin, soluble starch, and sucrose. Examples of the nitrogen sources include inorganic or organic materials such as ammonium salts, nitrate salts, corn steep liquor, peptone, casein, meat extract, soybean cake, and potato extract. Examples of the inorganic materials are calcium chloride, sodium dihydrogenphosphate, and magnesium chloride. In addition, yeast extracts, vitamins, and growth-stimulating factors may also be added to the medium. Preferred pH of the medium is about 5 to 8.

A preferred example of the medium for culturing Escherichia bacteria is M9 medium containing with glucose and casamino acids (Miller, Journal of Experiments in Molecular Genetics, 431-433, Cold Spring Harbor Laboratory, New York, 1972). If necessary, an agent for inducing the expression, such as 3β-indolylacrylic acid or isopropyl-beta-D-thiogalactopyranoside (IPTG) can be added to the medium thereby to increase the promoter efficiency. The transformant derived from an Escherichia bacterium host is usually cultured at about 15 to 43 °C for about 3 to 24 hours. If necessary, the culture may be aerated or agitated. The transformant derived from a Bacillus bacterium host is cultured usually at about 30 to 40 °C for about 6 to 24 hours. If necessary, the culture can be aerated or agitated.

As described above, the desired peptide or protein can be produced intracelluarly, in the cell membrane, or extracellularly by the transformant.

The desired peptide or protein can be isolated or purified from the culture described above, for example, by the following procedures.

To extract the desired peptide or protein from the cultured bacteria or cells, where appropriate, after the culture is completed, the bacteria or cells are collected by a well-known method and suspended in an appropriate buffer. The bacteria or cells are disrupted by such a method as ultrasonication, treatment with lysozyme and/or freeze-thaw cycling, and then subjected to the centrifugation or filtration to obtain a crude extract of the peptide or protein. The buffer may contain a protein denaturing agent such as urea or guanidine hydrochloride, or a surfactant such as Triton X-100™. When the peptide or protein is secreted into the culture broth, after the culture is completed, the supernatant can be separated and collected from the bacteria or cells, for example, by centrifugation.

The peptide or protein contained in the supernatant or the extract thus obtained can be purified by an appropriate combination of well-known isolation or purification methods. Such isolation or purification methods include a method utilizing difference in solubility such as salting out, solvent precipitation; a method mainly utilizing difference in molecular weight such as dialysis, ultrafiltration, gel filtration, SDS-polyacrylamide gel electrophoresis; a method utilizing difference in electric charge such as ion exchange chromatography; a method utilizing specific affinity such as affinity chromatography; a method utilizing difference in hydrophobicity such as reversed phase high performance liquid chromatography; a method utilizing difference in isoelectric point such as isoelectric focusing; and the like.

When the peptide or protein thus obtained is in a free form, it can be converted into a salt form by a well-known method or a variation thereof. On the other hand, when it is obtained in a salt form, it can be converted into the free form or a different salt form by a well-known method or a variation thereof.

Examples of the salt include a salt with an inorganic base, a salt with an organic base, a salt with an inorganic acid, a salt with an organic acid, a salt with a basic or acidic amino acid and so on.

Preferred examples of the salt with an inorganic base include an alkali metal salt such as sodium salt, potassium salt; alkali earth metal salt, such as calcium salt and magnesium salt; and aluminum salt, ammonium salt, etc.

Preferred examples of the salt with an organic base include salts with trimethylamine, triethylamine, pyridine, picoline, 2,6-lutidine, ethanolamine, diethanolamine, triethanolamine, cyclohexylamine, dicyclohexylamine, and N,N'-dibenzylethylenediamine, etc.

Preferred examples of the salt with an inorganic acid include salts with hydrochloric acid, hydrobromic acid, sulfuric acid, and phosphoric acid, etc.

Preferred examples of the salt with an organic acid include salts with formic acid, acetic acid, propionic acid, fumaric acid, oxalic acid, tartaric acid, maleic acid, citric acid, succinic acid, malic acid, methanesulfonic acid, benzenesulfonic acid, and benzoic acid, etc.

Preferred examples of the salt with a basic amino acid include salts with arginine, lysine, and ornithine, etc., and preferred examples of the salt with an acidic amino acid include salts with aspartic acid and glutamic acid, etc.

The peptide or protein produced by the recombinant can be treated, before or after the purification, with an appropriate protein-modifying enzyme so that the protein can be appropriately modified or deprived of a partial polypeptide. Examples of the protein-modifying enzyme include trypsin, chymotrypsin, arginyl endopeptidase, protein kinase, glycosidase and the like.

The thus produced peptide or protein, or a salt thereof can be detected using a specific antibody by an enzyme immunoassay and the like.

SEQ ID NOs in the sequence listing of the specification indicate the following sequences, respectively.

### [SEQ ID NO: 1]

This shows the nucleotide sequence of primer 1 used in Reference Example 1.

### [SEQ ID NO: 2]

This shows the nucleotide sequence of primer 2 used in Reference Example 1.

### [SEQ ID NO: 3]

This shows the nucleotide sequence of synthetic DNA (i) used in Reference Example 1.

### [SEQ ID NO: 4]

This shows the nucleotide sequence of synthetic DNA (ii) used in Reference Example 1.

### [SEQ ID NO: 5]

This shows the nucleotide sequence of synthetic DNA (iii) used in Reference Example 1.

### [SEQ ID NO: 6]

This shows the nucleotide sequence of synthetic DNA (iv) used in Reference Example 1.

### [SEQ ID NO: 7]

This shows the nucleotide sequence of synthetic DNA (v) used in Reference Example 2.

### [SEQ ID NO: 8]

This shows the nucleotide sequence of synthetic DNA (vi) used in Reference Example 2.

### [SEQ ID NO: 9]

This shows the nucleotide sequence of synthetic DNA 1 used in Example 1.

### [SEQ ID NO: 10]

This shows the nucleotide sequence of synthetic DNA 2 used in Example 1.

### [SEQ ID NO: 11]

This shows the nucleotide sequence of synthetic DNA 3 used in Example 1.

### [SEQ ID NO: 12]

This shows the nucleotide sequence of synthetic DNA 4 used in Example 1.

### [SEQ ID NO: 13]

This shows the nucleotide sequence of synthetic DNA 5 used in Example 2.

### [SEQ ID NO: 14]

This shows the nucleotide sequence of synthetic DNA 6 used in Example 2.

### [SEQ ID NO: 15]

This shows the nucleotide sequence of synthetic DNA 7 used in Example 3.

### [SEQ ID NO: 16]

This shows the nucleotide sequence of synthetic DNA 8 used in Example 3.

### [SEQ ID NO: 17]

This shows the nucleotide sequence of synthetic DNA 9 used in Example 4.

### [SEQ ID NO: 18]

This shows the nucleotide sequence of synthetic DNA 10 used in Example 4.

### [SEQ ID NO: 19]

This shows the nucleotide sequence of synthetic DNA 11 used in Example 6.

### [SEQ ID NO: 20]

This shows the nucleotide sequence of synthetic DNA 12 used in Example 6.

### [SEQ ID NO: 21]

This shows the nucleotide sequence of synthetic DNA 13 used in Example 6.

### [SEQ ID NO: 22]

This shows the nucleotide sequence of synthetic DNA 14 used in Example 6.

### [SEQ ID NO: 23]

This shows the nucleotide sequence of synthetic DNA 15 used in Example 7.

### [SEQ ID NO: 24]

This shows the nucleotide sequence of synthetic DNA 16 used in Example 7.

### [SEQ ID NO: 25]

This shows the nucleotide sequence of synthetic DNA 17 used in Example 7.

### [SEQ ID NO: 26]

This shows the nucleotide sequence of synthetic DNA 18 used in Example 7.

### [SEQ ID NO: 27]

This shows the nucleotide sequence of synthetic DNA 19 used in Example 7.

### [SEQ ID NO: 28]

This shows the nucleotide sequence of synthetic DNA 20 used in Example 7.

### [SEQ ID NO: 29]

This shows the nucleotide sequence of synthetic DNA 21 used in Example 7.

### [SEQ ID NO: 30]

This shows the nucleotide sequence of synthetic DNA 22 used in Example 8.

### [SEQ ID NO: 31]

This shows the nucleotide sequence of synthetic DNA 23 used in Example 8.

### [SEQ ID NO: 32]

This shows the nucleotide sequence of synthetic DNA 24 used in Example 8.

### [SEQ ID NO: 33]

This shows the nucleotide sequence of synthetic DNA 25 used in Example 8.

### [SEQ ID NO: 34]

This shows the nucleotide sequence positioned between -35 region and -10 region in the promoter of the invention.

### [SEQ ID NO: 35]

This shows the nucleotide sequence of NP3 promoter of the invention.

### [SEQ ID NO: 36]

This shows the nucleotide sequence of trp3 promoter of the invention.

### [SEQ ID NO: 37]

This shows the nucleotide sequence of -35 region

### [SEQ ID NO: 38]

This shows the nucleotide sequence of -10 region.

### [SEQ ID NO: 39]

This shows the nucleotide sequence of -10 region.

### EXAMPLES

The following examples will further illustrate the invention, but without the intention to limit the invention thereto.

The transformant Escherichia coli JM109/pGFPNP, obtained in Reference Example 1, is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry) located at Center No. 6, 1-1-1 Higasi, Tukuba-shi, Ibaraki 305-8566, Japan, under Accession Number FERM BP-7223 since July 17, 2000; and at Institute for Fermentation, Osaka (IFO) located at 2-17-85, Jyuso-Honmati, Yodogawa-ku, Osaka-shi, Osaka 532-8686, Japan, under Accession Number IFO 16426 since April 24, 2000.

The transformant Escherichia coli (MM294(DE3)/pTCHGH-Na), which contains plasmid pTCHGH-Na used in Example 8, is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry) located at Center No. 6, 1-1-1 Higasi, Tukuba-shi, Ibaraki 305-8566, Japan, under Accession Number FERM BP-6888 since December 10, 1997; and at Institute for Fermentation, Osaka (IFO) under Accession Number IFO 16124 since October 16, 1997.

The transformant Escherichia coli MM294/pNP3GHNO12, which contains plasmid pNP3GHNO12 used in Example 8, is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry) located at Center No. 6, 1-1-1 Higasi, Tukuba-shi, Ibaraki 305-8566, Japan, under Accession Number FERM BP-7611 since May 24, 2001; and at Institute for Fermentation, Osaka (IFO) under Accession Number IFO 16630 since May 17, 2001.

### Reference Example 1: Construction of Plasmid pGFPNP

### 1) Construction of Plasmid pGFPuvqc

In order to eliminate the NdeI restriction site present within the structural gene GFPuv in pGFPuv (Clontech), the histidine codon at position 77 was changed from CAT to CAC by site-directed mutagenesis (QuickChange™ Site Directed Mutagenesis Kit: Stratagene) using primer 1: CGTTATCCGGATCACATGAAACGGCATG (SEQ ID NO: 1) and primer 2: CATGCCGTTTCATGTGATCCGGATAACG (SEQ ID NO: 2), to thereby obtain plasmid pGFPuvqc.

### 2) Construction of Plasmid pGFPuvqd

Plasmid pGFPuvqc was digested with HindIII (Takara Shuzo) and KpnI (Takara Shuzo), followed by agarose gel electrophoresis. An approximately 3.3 kbp band was cut out, and the DNA was recovered using QIAquick Gel Extraction Kit (Qiagen). One hundred pmol each of synthetic DNA (i): AGCTTCATATGTTCGAAGTACTAGATCTGGTAC (SEQ ID NO: 3) and synthetic DNA (ii): CAGATCTAGTACTTCGAACATATGA (SEQ ID NO: 4) were dissolved in TE buffer, retained at 90°C for 10 min and then gradually cooled to room temperature for annealing. The thus annealed DNA fragment was inserted between the HindIII and KpnI sites in pGFPuvqc using Ligation Kit ver. 2 (Takara Shuzo), to thereby obtain plasmid pGFPuvqd.

### 3) Construction of Plasmid pNPHGH

Plasmid pNPHGH was constructed as described below, in which the T7 promoter of pTCHGH-Na described in Reference Example 1 of WO00/20439 is replaced with a synthetic promoter NP2. Briefly, plasmid pTCHGH-Na was digested with EcoRI (Takara Shuzo) and XbaI (Takara Shuzo), and then subjected to agarose gel electrophoresis. An approximately 4.6 kbp band was cut out from the gel, and the DNA was recovered using QIAquick Gel Extraction Kit (Qiagen).

One hundred pmol each of synthetic DNA (iii) comprising synthetic promoter NP2: AATTCTATAAAAATAATTGTTGACATATTTTATAAATTTTGGCATAATAGATCTAATTG TGAGCGGATAACAATTCTGCAGAAGCTTGAGCTCGGTACCCGGGGATCCT (SEQ ID NO: 5) and synthetic DNA (iv) comprising a nucleotide sequence complementary thereto: CTAGAGGATCCCCGGGTACCGAGCTCAAGCTTCTGCAGAATTGTTATCCGCTCACA ATTAGATCTATTATGCCAAAATTTATAAAATATGTCAACAATTATTTTTATAG (SEQ ID NO: 6) were dissolved in TE buffer, retained at 90°C for 10 min and then gradually cooled to room temperature for annealing. The resultant double-stranded DNA fragment was inserted between the EcoRI and XbaI sites in pTCHGH-Na using Ligation Kit ver. 2 (Takara Shuzo), to thereby obtain plasmid pNPHGH.

### 4) Construction of Plasmid pGFPNP

Plasmid pNPHGH was digested with BamHI (Takara Shuzo) and blunt-ended using DNA Blunting Kit (Takara Shuzo). The resultant DNA was further digested with NdeI and then subjected to agarose gel electrophoresis. An approximately 4.6 kbp band was cut out from the gel, and the DNA was recovered using QIAquick Gel Extraction Kit (Qiagen). Plasmid pGFPuvqd was digested with NdeI and StuI, and then subjected to agarose gel electrophoresis. An approximately 0.8 kbp band was cut out from the gel, and the DNA comprising GFPuv structural gene was recovered from the gel using QIAquick Gel Extraction Kit (Qiagen). The recovered DNA was inserted between the NdeI and BamHI (blunted) sites of pNPHGH using Ligation Kit ver. 2 (Takara Shuzo), to thereby obtain plasmid pGFPNP for selecting N-terminal optimized sequences.

Plasmid pGFPNP is an expression plasmid which comprises GFPuv structural gene to be transcribed by NP2 promoter and NdeI, Scal and AgeI restriction sites upstream of this structural gene. By utilizing these restriction sites, it is possible to insert a DNA fragment into this plasmid.

*Escherichia coli* JM109 was transformed with plasmid pGFPNP to thereby obtain *E. coli* JM109/pGFPNP.

### Reference Example 2: Construction of Plasmid pNPHGHlacts

Plasmid pNPHGH constructed in Reference Example 1 was digested with MscI, and the terminal sites were de-phosphorylated with Bacterial Alkaline Phosphatase (Nippon Gene). Plasmid pET11a was digested with NaeI and subjected to agarose gel electrophoresis. An approximately 1.6 kbp band was cut out from the gel, and the DNA was recovered using QIAquick Gel Extraction Kit (Qiagen). This DNA fragment comprising lacI was inserted into the MscI restriction site of pNPHGH using Ligation Kit ver. 2 (Takara Shuzo), to thereby obtain plasmid pNPHGHlac. Subsequently, the following procedures were carried out in order to replace the T7 terminator with a synthetic terminator. Plasmid pNPHGHlac was digested with EspI and PvuI, and then subjected to agarose gel electrophoresis. An approximately 6.1 kbp band was cut out from the gel, and the DNA was recovered using QIAquick Gel Extraction Kit (Qiagen).

One hundred pmol each of synthetic DNA (v): TGAGCATGCATACTAGTCTCGAGTAATCCCACAGCCGCCGCCAGTTCCGCTGGCGG CGGCATTTTCGAT (SEQ ID NO: 7) and synthetic DNA (vi): CGAAAATGCCGCCGCCAGCGGAACTGGCGGCGGCTGTGGGATTACTCGAGACTAG TATGCATGC (SEQ ID NO: 8) were dissolved in TE buffer, retained at 90°C for 10 min and then gradually cooled to room temperature for annealing. The resultant DNA was ligated between the EspI and PvuI sites in pNPHGHlac using Ligation Kit ver. 2 (Takara Shuzo), to thereby obtain plasmid pNPHGHlacts.

### Example 1: Construction of Plasmid pKMNP for Searching for High Expression Promoters

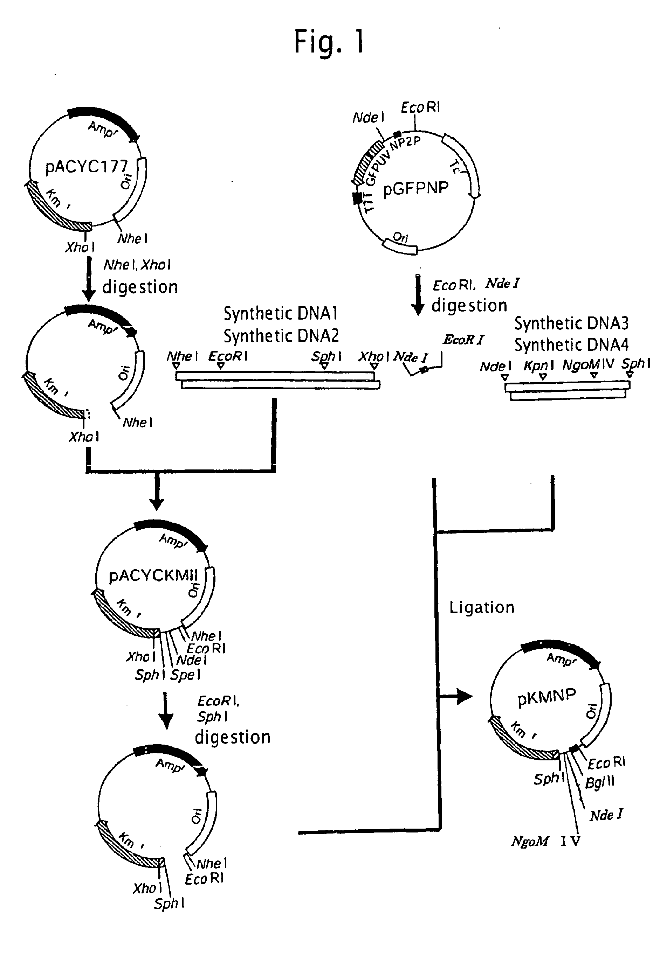

The search for high expression promoters was performed by primary selection using kanamycin resistance gene as a reporter and secondary selection using GFP (green fluorescent protein) as a reporter. Plasmid pKMNP used in the primary selection was constructed as described below (Fig. 1).

Plasmid pACYC177 (Wako Purechemical Industries) was digested with restriction enzymes NheI and XhoI, and then subjected to agarose gel electrophoresis. An approximately 3.6 kbp band was cut out from the gel, and the DNA was recovered using QIAquick Gel Extraction Kit (Qiagen).

One hundred pmol each of synthetic DNA 1: CTAGCGAATTCGAGCATATGAGCACTAGTGCATGCGAGCCATATTCAACGGGAAAC GTCTTGC (SEQ ID NO: 9) and synthetic DNA 2: TCGAGCAAGACGTTTCCCGTTGAATATGGCTCGCATGCACTAGTGCTCATATGCTC GAATTCG (SEQ ID NO: 10) were dissolved in TE buffer. Each 2 µl of synthetic DNA 1 and synthetic DNA 2 (0.1 mmole/L for each), 2 µl of ATP solution, 1 µl of T4 polynucleotide kinase, 2 µl of 10x reaction buffer attached to the kinase and 11 µl of distilled water were mixed at 37°C for 1 hr for phosphorylation. Then, the reaction solution was retained at 90°C for 10 min, followed by gradual cooling to room temperature for annealing. The thus annealed DNA fragment was inserted between the NheI and XhoI sites of pACYC177 using Ligation Kit ver. 2 (Takara Shuzo), to thereby obtain plasmid pACYCMII in which EcoRI and SphI restriction sites have been introduced upstream of the kanamycin resistance gene.

Plasmid pACYCMII was digested with EcoRI and SphI, and then subjected to agarose gel electrophoresis. An approximately 3.6 kbp band was cut out from the gel, and the DNA was recovered using QIAquick Gel Extraction Kit (Qiagen). Plasmid pGFPNP constructed in Reference Example 1 was digested with EcoRI and NdeI, and then subjected to agarose gel electrophoresis. An approximately 0.15 kbp band was cut out from the gel, and the DNA was recovered using QIAquick Gel Extraction Kit (Qiagen).

One hundred pmol each of synthetic DNA 3: TATGAGGGTACCGCCGGCTGCATG (SEQ ID NO: 11) and synthetic DNA 4: CAGCCGGCGGTACCCTCA (SEQ ID NO: 12) were dissolved in TE buffer. Each 2 µl of synthetic DNA 3 and synthetic DNA 4 (0.1 mmole/L for each), 2 µl of ATP solution, 1 µl of T4 polynucleotide kinase, 2 µl of 10x reaction buffer attached to the kinase and 11 µl of distilled water were mixed at 37°C for 1 hr for phosphorylation. Then, the reaction solution was retained at 90°C for 10 min, followed by gradual cooling to room temperature for annealing. The thus annealed DNA fragment and the EcoRI-NdeI fragment of pGFPNP were inserted between the EcoRI and SphI sites of pACYCMII using Ligation Kit ver. 2 (Takara Shuzo), to thereby obtain plasmid pKMNP.

This plasmid has a kanamycin resistance gene downstream of NP2 promoter (Fig. 8) functional in *E*. *coli*. By replacing this promoter region with a candidate promoter sequence comprising a random sequence between the -35 region and -10 region, it is possible to search for high expression promoters using kanamycin resistance as an indicator.

### Example 2: Construction of Plasmid pGFPNPv3 for Searching for High Expression Promoters

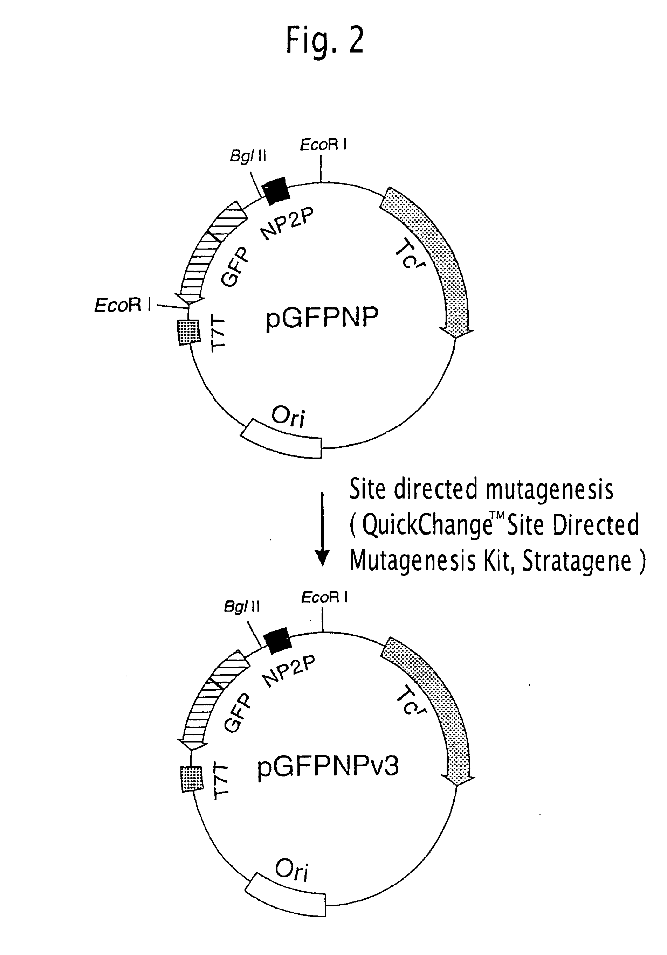

Plasmid pGFPNPv3 used for the secondary selection was constructed as described below (Fig. 2).

In order to eliminate one of the two EcoRI restriction sites present in pGFPNP constructed in Reference Example 1 which is located downstream of GFPuv structural gene, site-directed mutagenesis was performed on this plasmid with QuickChange Site Directed Mutagenesis Kit (Stratagene) using synthetic DNA 5: GATGAGCTCTACAAATAATAAATTCCAACTGAGCGC (SEQ ID NO: 13) and synthetic DNA 6: GCGCTCAGTTGGAATTTATTATTTGTAGAGCTCATC (SEQ ID NO: 14), to thereby obtain plasmid pGFPNPv3 in which the EcoRI restriction site downstream of the GFPuv structural gene has been eliminated.

Since this plasmid pGFPNPv3 has NdeI and EcoRI restriction sites upstream of GFPuv structural gene, it is possible to replace the NP2 promoter sequence upstream of the GFPuv with a candidate promoter sequence selected by primary selection using plasmid pKMRND constructed in Example 3 described later. Then, it becomes possible to measure the expression efficiency of a candidate promoter with the fluorescence intensity of GFPuv.

### Example 3: Primary Selection Using Kanamycin Resistance Gene as a Reporter

Plasmid pKMNP was digested with restriction enzymes BglII (Takara Shuzo) and EcoRI (Takara Shuzo), and then subjected to agarose gel electrophoresis to remove an approximately 50 bp band. The remaining DNA was purified using QIAquick Gel Extraction Kit (Qiagen). The resultant DNA fragment lacks the NP2 promoter region upstream of the kanamycin resistance gene.

Subsequently, a double-stranded DNA was prepared using *Z-Taq* DNA polymerase (Takara Shuzo), synthetic DNA 7: ACAATTAGATCTATTATGNNNNNNNNNNNNNNNNNTGTCAACAATTATTTTTATA GAATTCATCGATAAGCTT (SEQ ID NO: 15) as a template and synthetic DNA 8: AAGCTTATCGATGAATTC (SEQ ID NO: 16) as a primer. The extension reaction solution was prepared by mixing 10 µl of *Z-Taq* DNA polymerase, 50 µl of 10x reaction buffer attached to the polymerase, 80 µl of dNTP mixture, 3.6 µl of synthetic DNA 7 (141 µmol/L), 5 µl of synthetic DNA 8 (100 µmol/L) and 490 µl of distilled water. The extension reaction was performed at 95°C for 15 sec and at 68°C for 15 min. The resultant reaction solution was concentrated and desalted with Microcon 30 (Nippon Millipore), followed by digestion of the double-stranded DNA with BglII (Takara Shuzo) and EcoRI (Takara Shuzo). This digestion of the double-stranded DNA with restriction enzymes was performed as described below. Briefly, a reaction solution containing 25 µl of the concentrated and desalted reaction solution, 3 µl of buffer H (Takara Shuzo), and 1 µl each of BglII and EcoRI was retained at 37°C for 24 hrs to perform digestion. The resultant reaction solution was concentrated and desalted with Microcon 30 (Nippon Millipore) and then subjected to agarose gel electrophoresis. An approximately 50 bp band was cut out, and the DNA fragment was purified using QIAquick gel Extraction Kit (Qiagen). This DNA fragment was inserted between the BglII and EcoRI sites of pKMNP using Ligation Kit ver. 2 (Takara Shuzo).

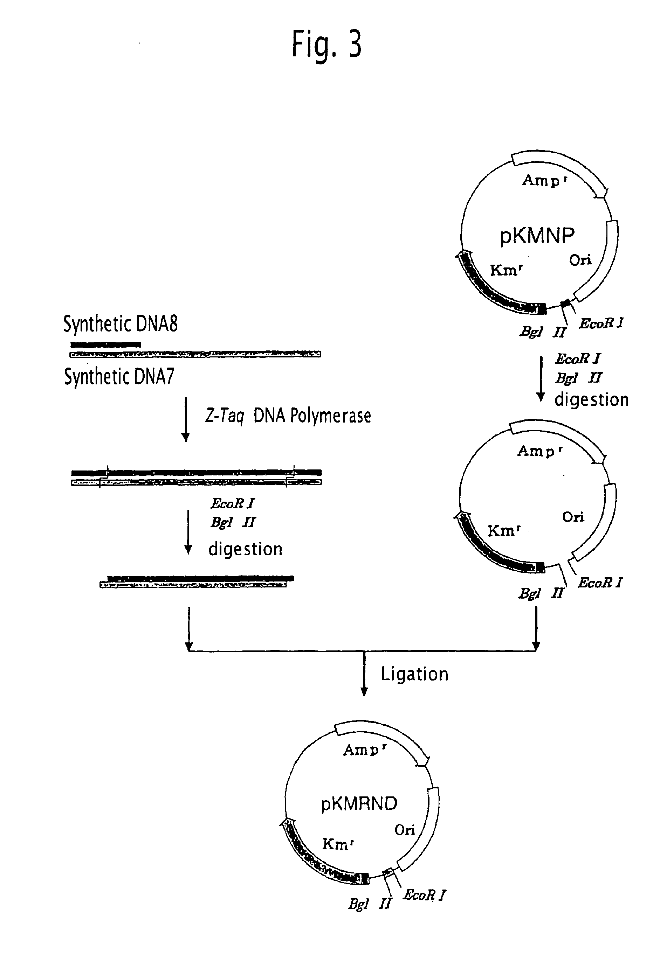

Then, *E*. *coli* MM294 was transformed with the resultant plasmid. Briefly, 10 µl of ligation solution was added to 100 µl of ice-cooled *E*. *coli* MM294 competent cells left for 30 min while ice-cooling. Subsequently, a heat-shock of 42°C for 45 sec was applied thereto. Then, 900 µl of SOC medium was added thereto, followed by shaking culture for 1 hr. The resultant culture broth was inoculated into kanamycin (100 mg/L)-containing LB medium. Cultivation in the kanamycin-containing medium allows selective growth of those clones having a plasmid in which a nucleotide sequence with a high promoter activity is inserted at the promoter region upstream of the kanamycin resistance gene. Cells were harvested after overnight culture at 37°C, and plasmids were purified from the cells using QIAprep Plasmid Kit (Qiagen). These plasmids (pKMRND) are plasmids in which candidate promoters having a random nucleotide sequence are inserted upstream of their kanamycin resistance gene (Fig. 3).

### Example 4: Secondary Selection Using GFP as a Reporter

Plasmid pGFPNPv3 was digested with restriction enzymes EcoRI (Takara Shuzo) and NdeI (Takara Shuzo), and then subjected to agarose gel electrophoresis to remove an approximately 150 bp band. The remaining DNA was purified using QIAquick Gel Extraction Kit (Qiagen) to thereby obtain a DNA fragment which lacks the promoter region upstream of the GFP gene.

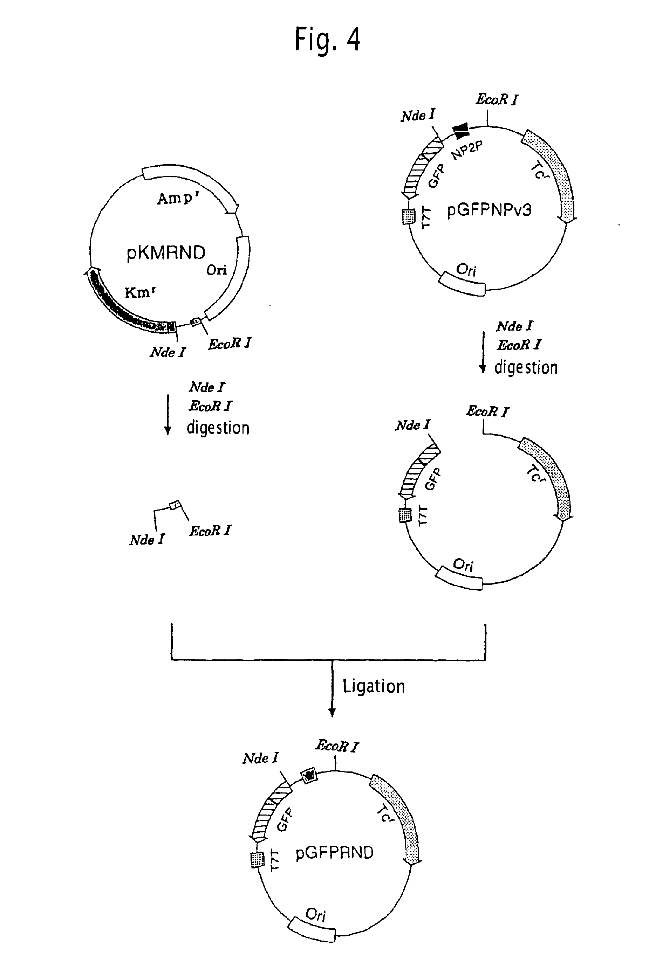

Plasmid pKMRND obtained in Example 3 was digested with restriction enzymes EcoRI (Takara Shuzo) and NdeI (Takara Shuzo), and then subjected to agarose gel electrophoresis. An approximately 150 bp band was cut out from the gel and purified using QIAquick Gel Extraction Kit (Qiagen) to thereby obtain a DNA fragment comprising a promoter region having a random sequence. This DNA fragment was inserted between the EcoRI and NdeI sites of pGFPNPv3 using Ligation Kit ver. 2 (Takara Shuzo) (Fig. 4).

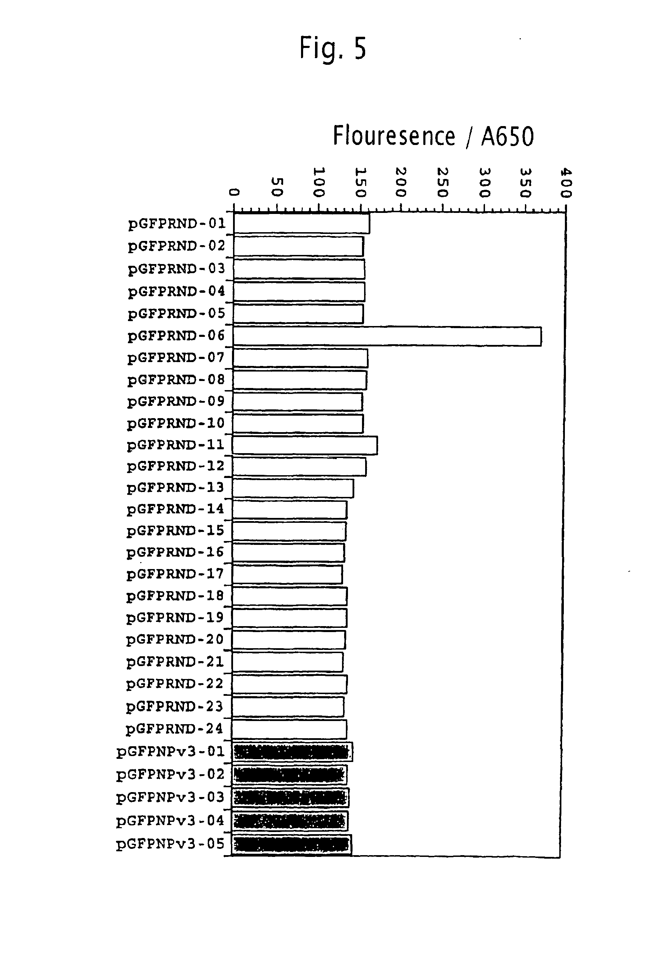

With the resultant plasmid, *E*. *coli* MM294 was transformed, then plated on LB agar medium containing 10 mg/L tetracycline and cultured overnight at 37°C to allow colony formation. The resultant colony was inoculated into 0.5 ml of LB medium containing 10 mg/L tetracycline. After overnight culture at 37°C, 50 ml of the culture broth of each colony was transferred into a 96-well microplate in which 150 ml of physiological saline had been dispensed. Then, absorbance at 650 nm (A₆₅₀) was measured with a 96 microplate reader (Nippon Molecular Device), and fluorescence intensity with the excitation wavelength of 355 nm was measured at a wavelength of 538 nm with a multi-plate fluorescence measuring apparatus (Dainippon Pharmaceutical). The ratio of the fluorescence intensity to the absorbance was shown as GFP expression efficiency (Fig. 5).

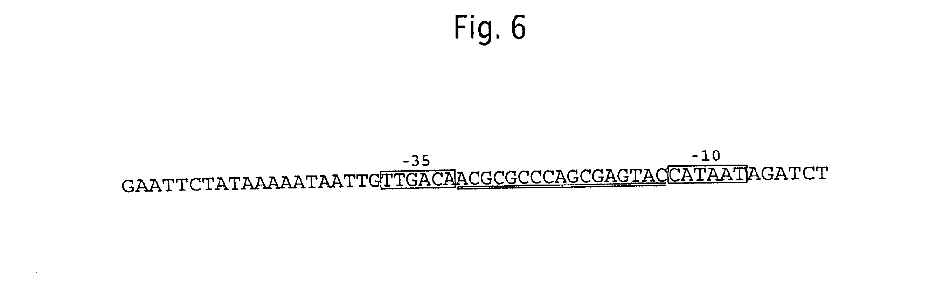

For each colony, a colony PCR was performed using synthetic DNA 9: TTCATACACGGTGCCTGACTGCGTTAG (SEQ ID NO: 17) and synthetic DNA 10: TCAACAAGAATTGGGACAACTCC (SEQ ID NO: 18), followed by agarose gel electrophoresis to confirm the insertion of the DNA fragment of interest. Further, the colony PCR product was purified using QIAquick PCR Purification Kit (Qiagen), and the nucleotide sequence thereof was determined using synthetic DNAs 9 and 10. The results revealed that pGFPRND No. 6 showing the highest GFP expression efficiency had the sequence as shown in Fig. 6 upstream of the GFP gene. In this Figure, the portion indicated with underline (the nucleotide sequence represented by SEQ ID NO: 34) is the region having a random sequence. The promoter sequence obtained here is designated "NP3 promoter'' in the present specification (Fig. 8).

### Example 5: Comparison of Promoter Activities in different E. coli Strains

With GFP expression plasmids: pGFPRND No. 6 having the high expression promoter (NP3) obtained in Example 4 and pGFPNPv3 having NP2 promoter, *E*. *coli* strains MM294, JM109, HB101 and DH5 were transformed. The resultant cells were plated on LB agar medium containing 10 mg/L tetracycline and cultured overnight at 37°C to allow colony formation. From the resultant colonies, 10 clones of each strain were taken at random and inoculated individually into 0.5 ml of LB medium containing 10 mg/L tetracycline. After overnight culture at 37°C, 50 ml of the culture broth of each colony was transferred into a 96-well microplate in which 150 ml of physiological saline had been dispensed. Then, absorbance at 650 nm (A₆₅₀) was measured with a 96 microplate reader (Nippon Molecular Device), and fluorescence intensity with the excitation wavelength of 355 nm was measured at a wavelength of 538 nm with a multi-plate fluorescence measuring apparatus (Dainippon Pharmaceutical). The ratio of the fluorescence intensity to the absorbance was shown as GFP expression efficiency.

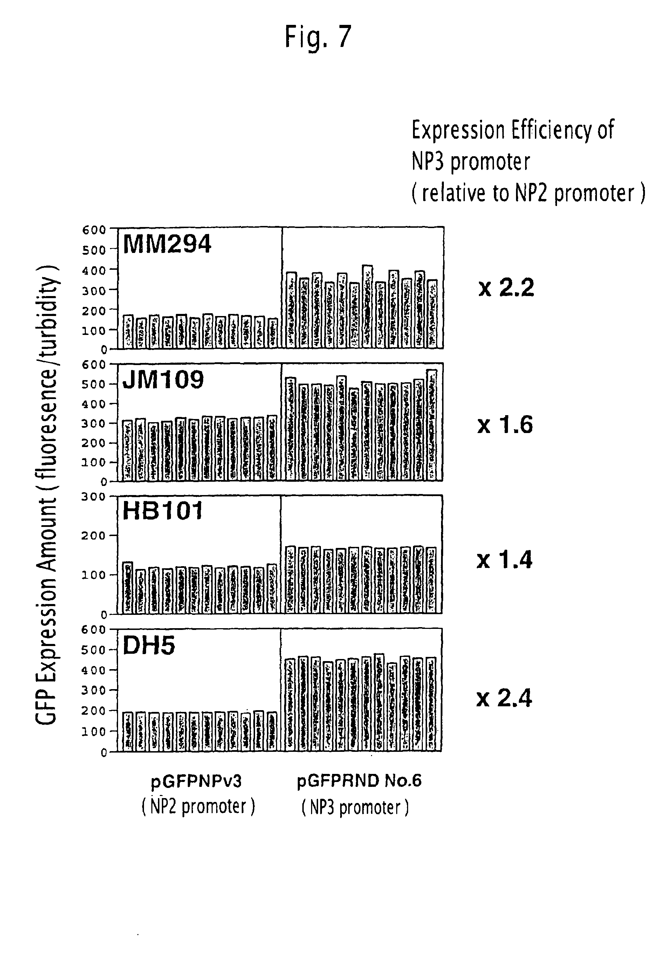

As shown in Fig. 7, NP3 promoter selected in Example 4 exhibited 1.4- to 2.4-fold higher expression efficiency in all the *E. coli* strains used (MM294, JM109, HB101 and DH5) than NP2 promoter (Fig. 7). It has been revealed that the sequence found in Example 4 has a high promoter activity regardless of the kind of *E*. *coli* host.

### Example 6: Expression Activity when Other -10 Sequences Are Used

As shown in Fig. 8, NP3 promoter is characterized by the nucleotide sequence in the intervening region between the -35 sequence and the -10 sequence of NP2 promoter. In order to determine whether this nucleotide sequence can show the high expression activity regardless of the kind of nearby sequences in the promoter (e.g. the -10 sequence), the inventors constructed a promoter which has the sequence obtained in Example 4 between the -35 sequence and the -10 sequence both used in trp promoter. This promoter was designated "trp3". An expression plasmid having trp or trp3 promoter upstream of GFP structural gene was constructed as described below.

Plasmid pGFPNPv3 obtained in Example 2 was digested with restriction enzymed EcoRI (Takara Shuzo) and BglII (Takara Shuzo), and subjected to agarose gel electrophoresis. An approximately 4.9 kbp band was cut out from the gel and purified with QIAquick Gel Extraction Kit (Qiagen) to thereby obtain a DNA fragment which lacks the promoter region upstream of GFP gene.

Trp promoter was constructed from synthetic DNA 11: AATTCTATAAAAATAATTGTTGACAATTAATCATCGGCTCGTATAATA (SEQ ID NO: 19) and synthetic DNA 12: GATCTATTATACGAGCCGATGATTAATTGTCAACAATTATTTTTATAG (SEQ ID NO: 20), and trp3 promoter was constructed from synthetic DNA 13: AATTCTATAAAAATAATTGTTGACAACGCGCCCAGCGAGTACTATAATA (SEQ ID NO: 21) and synthetic DNA 14: GATATTTTTATTAACAACTGTTGCGCGGGTCGCTCATGATATTATCTAG (SEQ ID NO: 22). Briefly, 2 µl each of synthetic DNA 11 and synthetic DNA 12, or synthetic DNA 13 and synthetic DNA 14 (0.1 mmole/L for each), 2 µl of ATP solution, 1 µl of T4 polynucleotide kinase, 2 µl of 10x reaction buffer attached to the kinase and 11 µl of distilled water were mixed and reacted at 37°C for 1 hr for phosphorylation. The reaction solution was retained at 95°C for 10 min, and then gradually cooled to room temperature for annealing. The thus annealed DNA fragment was inserted between the EcoRI and BglII sites of plasmid pGFPNPv3 using Ligation Kit ver. 2 (Takara Shuzo).

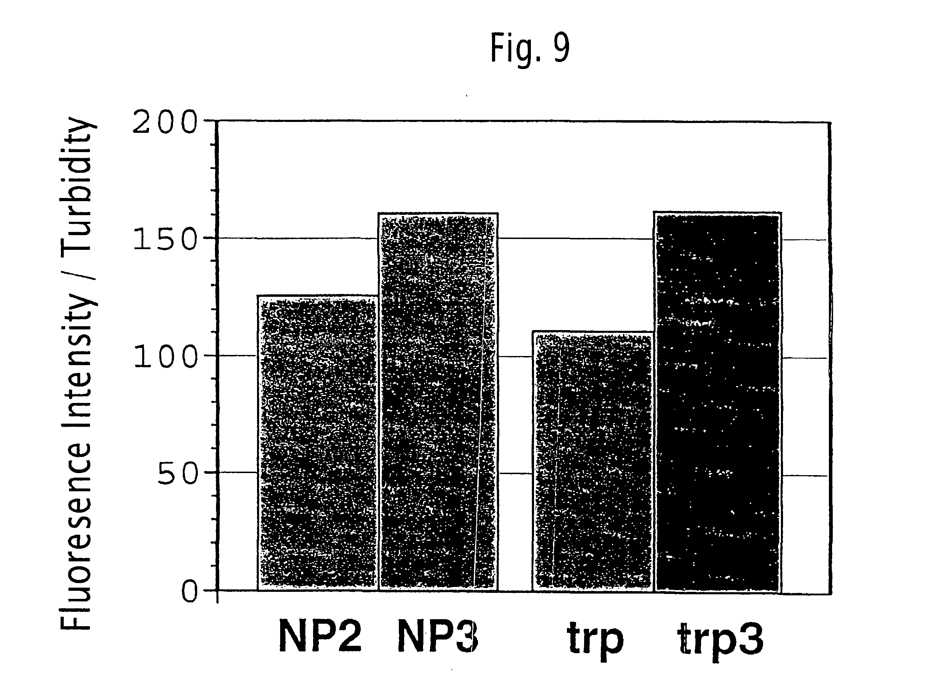

With the resultant plasmid, *E*. *coli* JM109 was transformed, then plated on LB agar medium containing 10 mg/L tetracycline and cultured overnight at 37°C to allow colony formation. The resultant colony was inoculated into 0.5 ml of LB medium containing 10 mg/L tetracycline. After overnight culture at 37°C, 50 ml of the culture broth of each colony was transferred into a 96-well microplate in which 150 ml of physiological saline had been dispensed. Then, absorbance at 650 nm (A₆₅₀) was measured with a 96 microplate reader (Nippon Molecular Device), and fluorescence intensity with the excitation wavelength of 355 nm was measured at a wavelength of 538 nm with a multi-plate fluorescence measuring apparatus (Dainippon Pharmaceutical). The ratio of the fluorescence intensity to the absorbance was shown as GFP expression efficiency (Fig. 9).

As a result, trp3 promoter which has the sequence obtained in Example 4 between the -35 region and the -10 region of trp promoter exhibited higher expression efficiency than trp promoter. From this, it is believed that a promoter having the sequence found in Example 4 between the -35 region and the -10 region has high expression efficiency regardless of the kind of nucleotide sequences of the -35 region and the -10 region.

### Example 7: Optimization of Nucleotide Sequence encoding N-Terminal of Human Growth Hormone

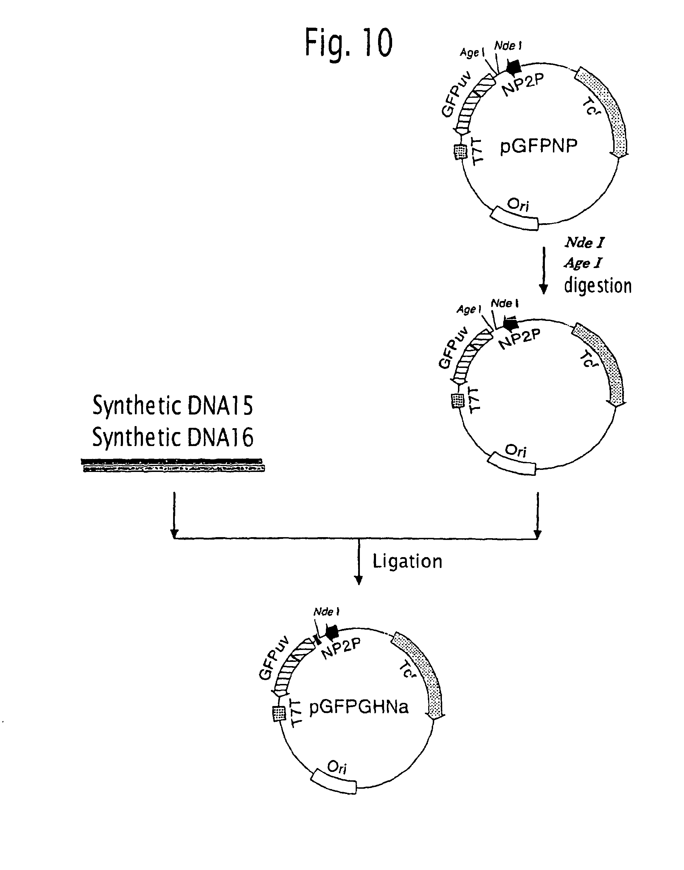

The nucleotide sequence encoding an N-terminal portion of human growth hormone which is optimal for expression was selected.
1) pGFPNP was digested with restriction enzymes AgeI (Nippon Gene) and NdeI (Takara Shuzo), and subjected to agarose gel electrophoresis. An approximately 4.9 kbp band was cut out from the gel and purified with QIAquick Gel Extraction Kit (Qiagen) to thereby obtain a DNA fragment.
   In order to phosphorylate the 5' end of synthetic DNA 15: TATGTTCCCAACCATTCCCTTATCCAGGCTTTTTGACAACGCTTTA (SEQ ID NO: 23) and synthetic DNA 16: CCGGTAAAGCGTTGTCAAAAAGCCTGGATAAGGGAATGGTTGGGAACA (SEQ ID NO: 24), 2 µl of synthetic DNA 15 or synthetic DNA 16 (0.1 mmol/L), 2 µl of T4 polynucleotide kinase (Nippon Gene), 2 µl of 10x Reaction buffer attached to the kinase, 2 µl of ATP, and 13 µl of distilled water were mixed and reacted at 37°C for 1 hr. The reaction solution was retained at 96°C for 10 min, and then gradually cooled to room temperature to allow formation of a double strand. The resultant DNA fragment was inserted between the AgeI and NdeI sites of pGFPNP using Ligation Kit ver. 2 (Takara Shuzo) to thereby obtain expression plasmid pGFPGHNa, which has a wild-type nucleotide sequence encoding the N-terminal portion of human growth hormone upstream of the GFP gene (Fig. 10).
2) pGFPNP was digested with restriction enzymes AgeI (Nippon Gene) and NdeI (Takara Shuzo), and subjected to agarose gel electrophoresis. An approximately 4.9 kbp band was cut out from the gel and purified with QIAquick Gel Extraction Kit (Qiagen) to thereby obtain a DNA fragment.

Random nucleotide sequences encoding the N-terminal amino acids of human growth hormone were prepared as described below. Briefly, using mixed base-containing synthetic DNA 17: TCAAGAGTTTACCGGTAAAGCGTTGTCAAAAAGCCTNGANAGNGGDATNGTNGGR AACATATGTTGGCGCGCCTT (SEQ ID NO: 25), synthetic DNA 18: TCAAGAGTTTACCGGTAAAGCGTTGTCAAAAAGCCTRCTNAGNGGDATNGTNGGR AACATATGTTGGCGCGCCTT (SEQ ID NO: 26), synthetic DNA 19: TCAAGAGTTTACCGGTAAAGCGTTGTCAAAAAGCCTNGAYAANGGDATNGTNGGR AACATATGTTGGCGCGCCTT (SEQ ID NO: 27) and synthetic DNA 20: TCAAGAGTTTACCGGTAAAGCGTTGTCAAAAAGCCTRCTYAANGGDATNGTNGGR AACATATGTTGGCGCGCCTT (SEQ ID NO: 28) as a template and using synthetic DNA 21: AAGGCGCGCCAACATATG (SEQ ID NO: 29) as a primer, double-stranded DNAs were prepared with *Z-Taq* DNA polymerase (Takara Shuzo). Each 5 µl. The extension reaction solution was prepared by mixing 4 µl of *Z-Taq* DNA polymerase, 10 µl of 10x Reaction buffer attached to the polymerase, 16 µl of dNTP mixture, 2 µl each of synthetic DNA 17 (44.4 µmol/L), synthetic DNA 18 (22.2 µmol/L), synthetic DNA 19 (22.2 µmol/L) and synthetic DNA 20 (11.1 µmol/L), 3 µl of synthetic DNA 21 (100 µmol/L) and 56 µl of distilled water. The extension reaction was performed at 95°C for 5 sec, at 55°C for 5 sec, and at 72°C for 10 min. The resultant reaction solution was concentrated and desalted using Microcon 30 (Nippon Millipore) and then treated with AgeI (Nippon gene) and NdeI (Takara Shuzo) for cutting the resultant double-stranded DNA. From the thus treated reaction solution, a DNA fragment was purified using QIAquick Gel Extraction Kit (Qiagen).

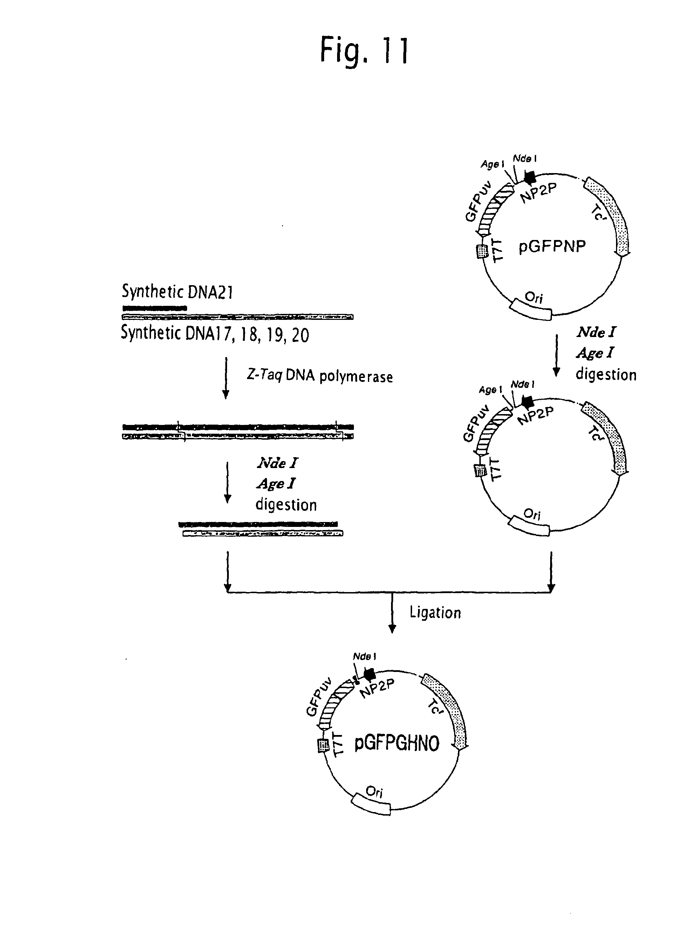

This DNA fragment was inserted between the NdeI and AgeI sites of pGFPNP using Ligation Kit ver. 2 (Takara Shuzo) to thereby construct expression plasmid pGFPGHNO, which has a random nucleotide sequence encoding the N-terminal amino acids of human growth hormone at the N-terminal portion of the GFP (Fig. 11).

With plasmid pGFPGHNa or pGFPGHNO, *E*. *coli* MM294 was transformed, then plated on LB agar medium containing 10 mg/L tetracycline and cultured overnight at 37°C to allow colony formation. The resultant colony was inoculated into 0.5 ml of LB medium containing 10 mg/L tetracycline. After overnight culture at 37°C, 50 ml of the culture broth of each colony was transferred into a 96-well microplate in which 150 ml of physiological saline had been dispensed. Then, absorbance at 650 nm (A₆₅₀) was measured with a 96 microplate reader (Nippon Molecular Device), and fluorescence intensity with the excitation wavelength of 355 nm was measured at a wavelength of 538 nm with a multi-plate fluorescence measuring apparatus (Dainippon Pharmaceutical). The ratio of the fluorescence intensity to the absorbance was shown as GFP expression efficiency.

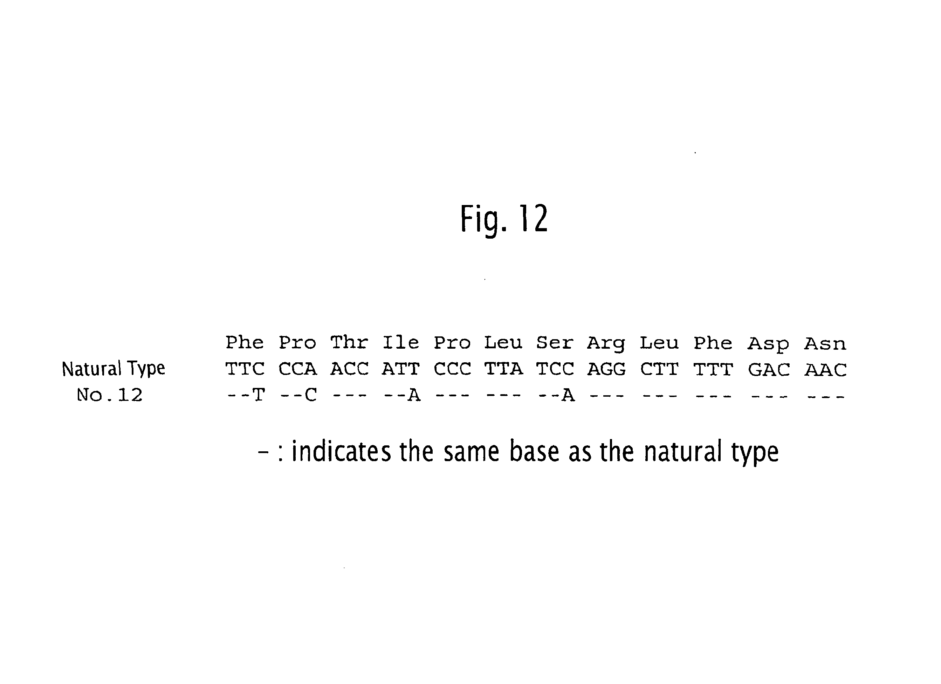

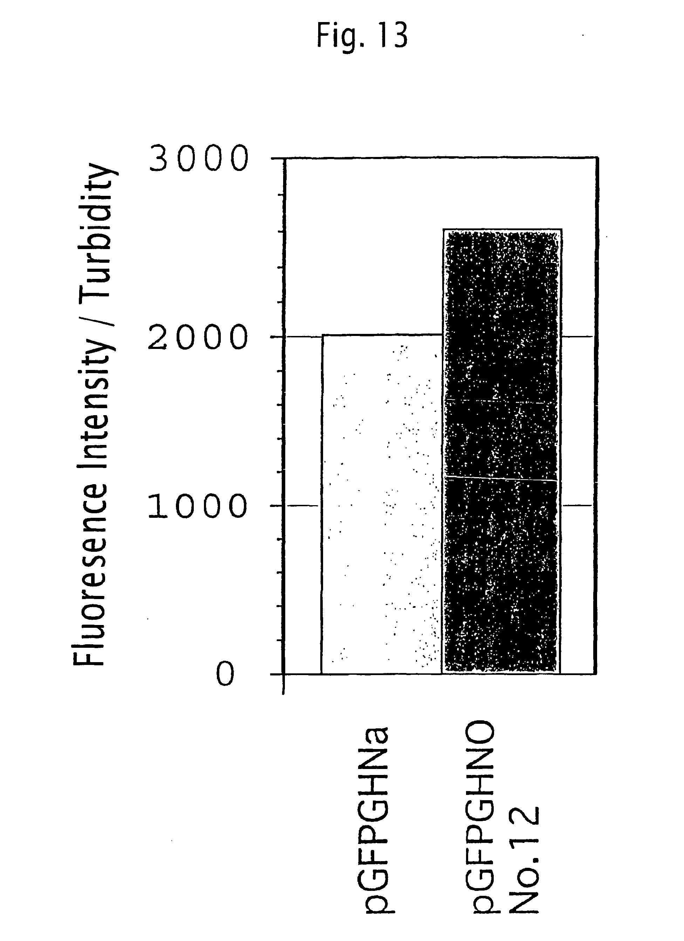

As a result, the GFP expression efficiency of pGFPGHNO clone No. 12 was about 20% higher than that of pGFPGHNa (Fig. 13). It was revealed that the nucleotide sequence of pGFPGHNO clone No. 12 had four bases substituted relative to the wild-type nucleotide sequence (Fig. 12).

### Example 8: Construction of Human Growth Hormone Expression Clones Using T7 Promoter

Human growth hormone expression plasmid pTCHGH-Na using T7 promoter as described in WO00/20439 was digested with restriction enzymes AgeI (Nippon Gene) and NdeI (Takara Shuzo) and then subjected to agarose gel electrophoresis. An approximately 4.9 kbp band was cut out from the gel and purified with QlAquick Gel Extraction Kit (Qiagen) to thereby obtain a DNA fragment.

A PCR reaction was performed with *Pfu* DNA polymerase (Takara Shuzo) using plasmid pTCHGH-Na as a template and synthetic DNA 22: ATATGCTAAGAGCCCATCGTCTGCACCAGC (SEQ ID NO: 30) and synthetic DNA 23: CTGTAGGTCTGCTTGAAGATCTGC (SEQ ID NO: 31). The PCR reaction solution was prepared by mixing 2 µl *of Pfu* DNA polymerase, 10 µl of 10x Reaction. buffer attached to the polymerase, 10 µl of dNTP mixture, 0.2 µl of plasmid pTCHGH-Na (157 ng/µl), 1 µl each of synthetic DNA 22 and synthetic DNA 23 (100 µmol/L for each) and 75.2 µl of distilled water. The PCR reaction was performed with 25 cycles of reactions at 98°C for 1 sec, at 55°C for 3 sec and at 72°C for 7 sec. After the PCR, the amplified DNA fragment was purified with QIAquick PCR Purification Kit. The DNA fragment was digested with restriction enzymes BstXI (Takara Shuzo) and BanII (Takara Shuzo) and then subjected to agarose gel electrophoresis. An approximately 0.25 kbp band was cut out and purified with QIAquick Gel Extraction Kit (Qiagen) to obtain a DNA fragment.

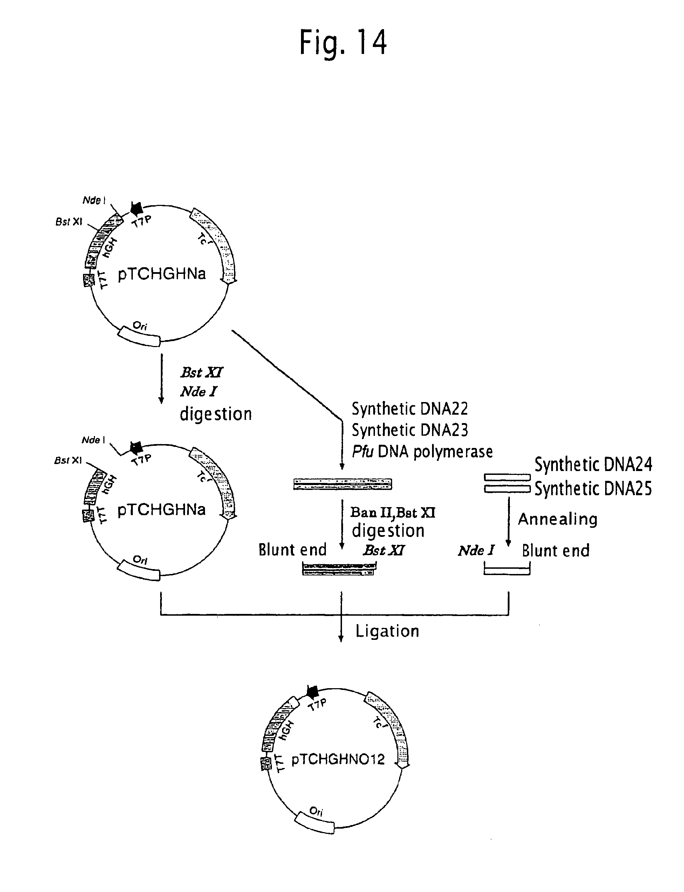

In order to phosphorylate the 5' end of synthetic DNA 24: TATGTTTCCCACCATACCCTTATCAAGGCTTTTTG (SEQ ID NO: 32) and synthetic DNA 25: CAAAAAGCCTTGATAAGGGTATGGTGGGAAACA (SEQ ID NO: 33), 2 µl each of synthetic DNA 24 and synthetic DNA 25 (0.1 mmol/L), 1 µl of T4 polynucleotide kinase (Nippon Gene), 2 µl of 10x Reaction buffer attached to the kinase, 2 µl of ATP, and 11 µl of distilled water were mixed and reacted at 37°C for 1 hr. The reaction solution was retained at 96°C for 10 min, and then gradually cooled to room temperature to allow formation of a double strand. The resultant DNA fragment and the BanII-BstXI fragment of the above-obtained PCR product were inserted between the NdeI and BstXI sites of pTCHGH-Na using Ligation Kit ver. 2 (Takara Shuzo), to thereby obtain human growth hormone expression plasmid pTCHGHNO12, which has the optimized nucleotide sequence encoding the N-terminal downstream of T7 promoter (Fig. 14).

Subsequently, human growth hormone expression plasmid pNPHGHNO12 which has the optimized nucleotide sequence encoding the N-terminal downstream of NP2 promoter was constructed as described below.

Plasmid pNPHGHlacts constructed in Reference Example 2 was digested with restriction enzymes NdeI (Takara Shuzo) and Bpul102I (Takara Shuzo), and then subjected to agarose gel electrophoresis. An approximately 5.5 kbp band was cut out from the gel and purified with QIAquick Gel Extraction Kit (Qiagen) to thereby obtain a DNA fragment.

Plasmid pTCHGHNO12 constructed in Reference Example 2 was digested with restriction enzymes NdeI (Takara Shuzo) and Bpu1102I (Takara Shuzo), and then subjected to agarose gel electrophoresis. An approximately 0.6 kbp band was cut out from the gel and purified with QIAquick Gel Extraction Kit (Qiagen) to thereby obtain a DNA fragment.

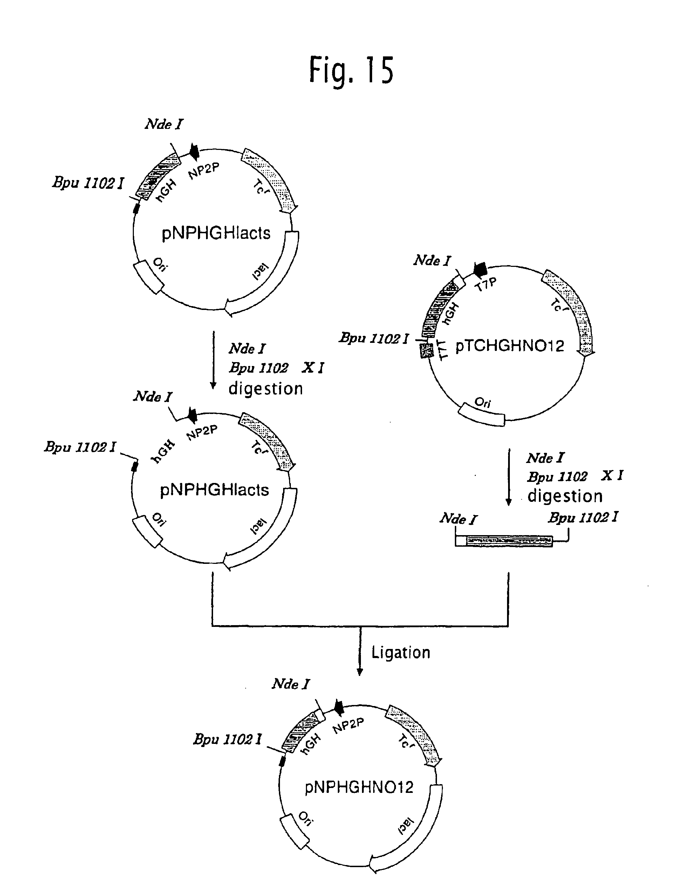

This DNA fragment was inserted between the NdeI and Bpu1102I sites of plasmid pNPHGHlacts using Ligation Kit ver. 2 (Takara Shuzo) to thereby obtain expression plasmid pNPHGHNO12 (Fig. 15).

Plasmid pNPHGHNO12 was digested with restriction enzymes NheI (Takara Shuzo) and NdeI (Takara Shuzo), and then subjected to agarose gel electrophoresis. An approximately 5.8 kbp band was cut out from the gel and purified with QIAquick Gel Extraction Kit (Qiagen) to thereby obtain a DNA fragment.

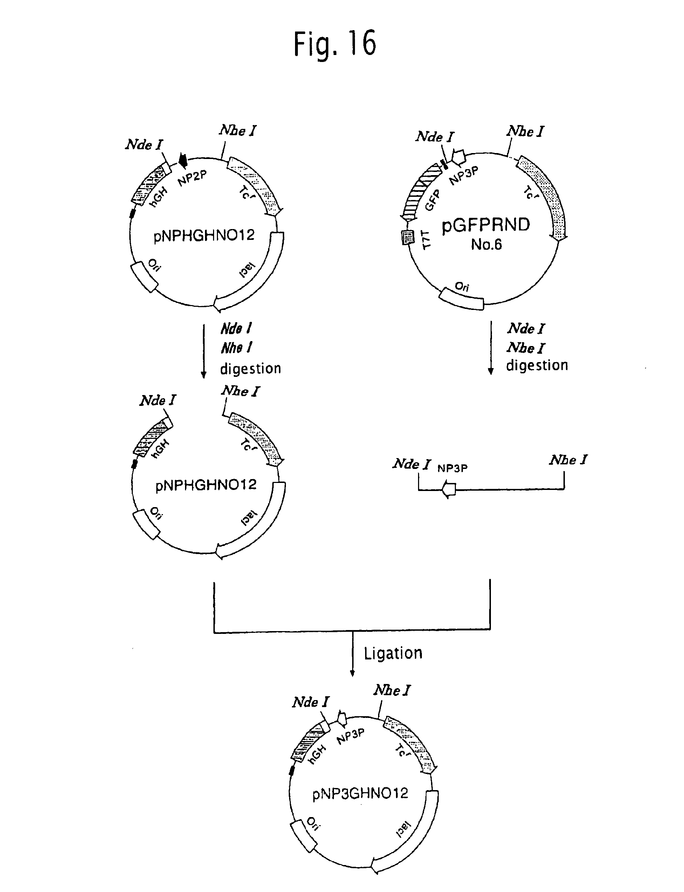

Plasmid pGFPRND No. 6 obtained in Example 4 was digested with restriction enzymes NheI (Takara Shuzo) and NdeI (Takara Shuzo), and then subjected to agarose gel electrophoresis. An approximately 0.4 kbp band was cut out from the gel and purified with QIAquick Gel Extraction Kit (Qiagen) to thereby obtain a DNA fragment. This fragment was inserted between the NheI and NdeI sites of pNPHGHNO12 using Ligation Kit ver. 2 (Takara Shuzo) to thereby obtain expression plasmid pNP3GHNO12 (Fig. 16).

### Example 9: Construction of Human Growth Hormone Expression Clones Using NP3 Promoter

*E*. *coli* MM294 (DE3) was transformed with plasmid pTCHGH-Na to thereby obtain human growth hormone expression clone MM294 (DE3)/pTCHGH-Na which uses T7 promoter.

*E*. *coli* MM294 was transformed with plasmid pNP3GHNO12 to thereby obtain human growth hormone expression clone MM294/pNP3GHNO12 which uses NP3 promoter.

### Example 10: Expression of Human Growth Hormone Using NP3 Promoter: 1

Human growth hormone expression clone MM294 (DE3)/pTCHGH-Na using T7 promoter and human growth hormone expression clone MM294/pNP3GHNO12 using NP3 promoter both obtained in Example 9 were cultured separately in 1 liter of 10 mg/L tetracycline-containing LB medium (1% peptone, 0.5% yeast extract, 0.5% sodium chloride) at 30°C for 16 hrs. The resultant culture broth (75 ml each) was transferred into a 3-liter jar fermentor containing 1.5 liters of primary fermentation medium (1.68% sodium monohydrogenphosphate, 0.3% sodium dihydrogenphosphate, 0.1 % ammonium chloride, 0.05% sodium chloride, 0.024% magnesium sulfate, 0.02% New Pole LB-625, 0.0005% thiamine hydrochloride, 1.5% glucose, 1.0% casamino acid, 1.0% yeast extract) and cultured at 37°C under aeration at 16 L/min and agitation at 200 rpm. When the turbidity of the culture broth reached about 1200 Klett units for MM294 (DE3)/pTCHGH-Na clone and about 600 Klett units for MM294/pNP3GHNO12 clone, isopropyl-β-D-thiogalactopyranoside (IPTG) was added to give a concentration of 0.075 mmole/L. From 4 hrs after the start of the cultivation, 20 g of glucose was added per hour. The cultivation was continued up to 18 hrs after the start of cultivation.

A 0.2 ml sample was taken from the culture broth at regular intervals and centrifuged at 12000 rpm for 10 min. The supernatant was discarded, and the resultant cells were subjected to ELISA to determine the expression level of human growth hormone. To cells obtained from 0.2 ml of the culture broth; 0.5 ml of 0.1 mol/L Tris buffer (pH 7.0) was added and suspended. This suspension (0.1 ml) was added to 0.4 ml of extraction buffer (2.5% sodium dodecyl sulfate-containing 0.1 mol/L Tris buffer) and agitated well, and then disrupted by sonication at 4°C for 5 min using Bioruptor (Olympus). After the disruption by sonication, the suspension was centrifuged at 13,000 rpm at 4°C for 10 min. The resultant supernatant was diluted 20603-fold with a dilution buffer (0.02 mol/L phosphate buffer, 0.14 mol/L sodium chloride, 10% BlockAce (Snow Brand Milk Products), 0.1% Tween 20, 0.02% Merthiolate).

This dilution (0.1 ml) was added to a 96-well microwell plate pre-coated with 5 µg/ml of anti-human growth hormone monoclonal antibody and left at room temperature for 2 hrs. Subsequently, each well was washed with a washing buffer (0.02 ml/L phosphate buffer, 0.14 mol/L sodium chloride, 0.1% Tween 20). Then, biotinylated rabbit anti-human growth hormone polyclonal antibody (5 µg/ml) was added thereto and left at room temperature for 1 hr. Each well was washed sufficiently with the washing buffer, and then 500-fold dilution of alkali phosphatase-streptavidin solution (Vector) was added thereto and left at room temperature for 30 min. Each well was again washed with the washing buffer sufficiently, and then the alkali phosphatase activity thereof was determined using BluePhos Microwell Phosphatase Substrate System.

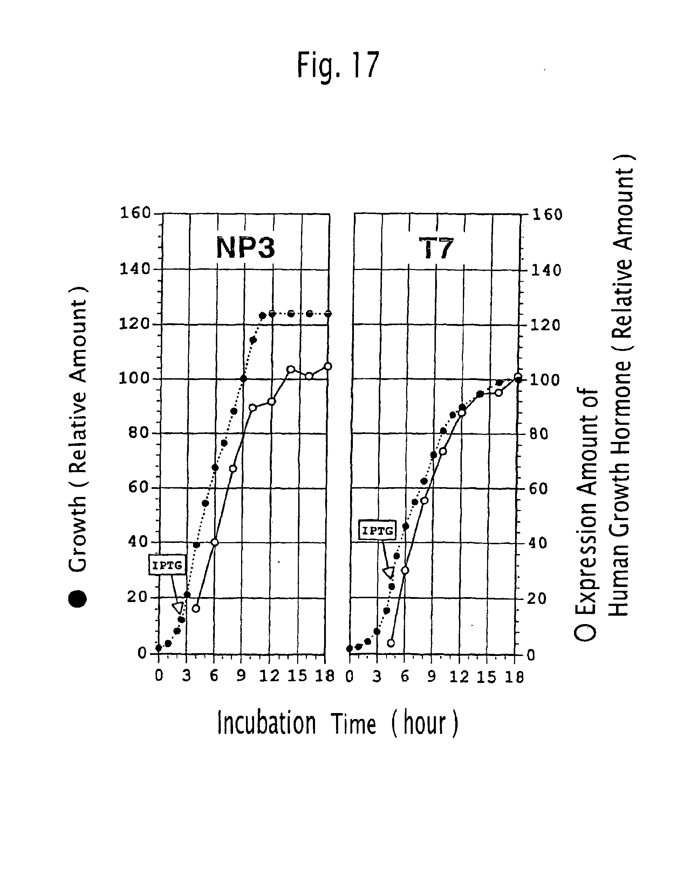

Expression levels of human growth hormone are shown in relative values taking the maximum expression level per culture broth of human growth hormone expression clone MM294 (DE3)/pTCHGH-Na as 100 (Fig. 17).

### Example 11: Expression of Human Growth Hormone Using NP3 Promoter: 2

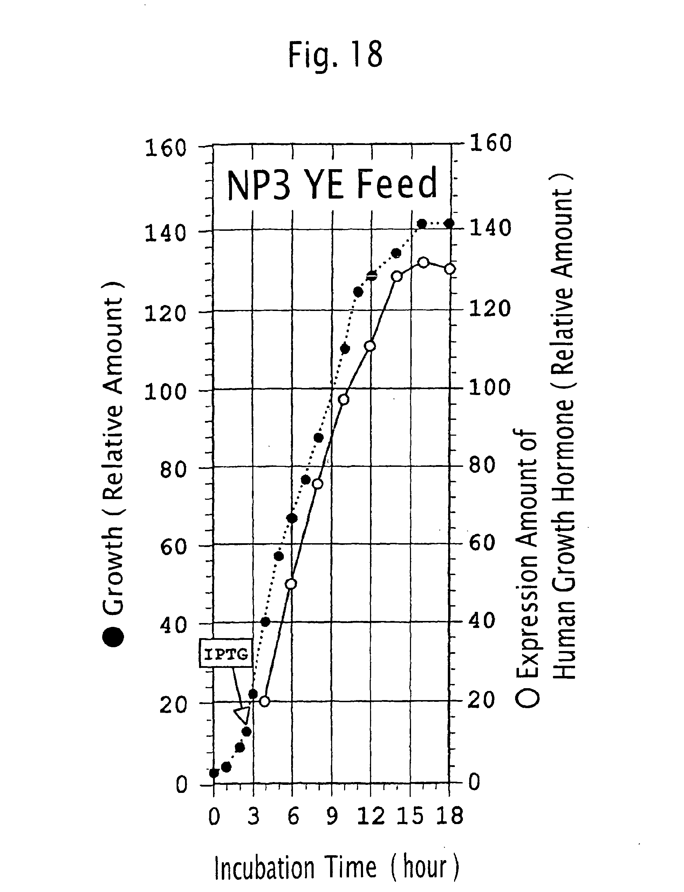

The expression of human growth hormone expression clone MM294/pNP3GHNO12 using NP3 promoter was tested in the same manner as in Example 10, except that yeast extract was supplied 4, 5, 7 and 8 hrs after the start of the cultivation (0.35% each time). Expression levels of human growth hormone were determined in the same manner as in Example 10 and shown in relative values taking the maximum expression level per culture broth of MM294 (DE3)/pTCHGH-Na clone in Example 10 as 100. While MM294 (DE3)/pTCHGH-Na clone did not show increase in the expression level even when yeast extract was supplied additionally, the expression level in MM294/pNP3GHNO12 clone increased up to 1.3-fold when yeast extract was supplied additionally (Fig. 18).

### INDUSTRIAL APPLICABILITY

The DNA comprising the NP3 promoter of the invention has an excellent promoter activity, and thus a target peptide or protein can be produced efficiently in large amounts through linking the structural gene encoding the target peptide or protein downstream of the promoter.

## Claims

1. A promoter comprising the same or substantially the same nucleotide sequence as that shown by SEQ ID NO: 34.

2. The promoter according to claim 1, which comprises the same or substantially the same nucleotide sequence as that shown by SEQ ID NO: 34 between any type of -35 region and any type of -10 region.

3. The promoter according to claim 2, in which the -35 region has the nucleotide sequence shown by SEQ ID NO: 37.

4. The promoter according to claim 2, in which the -10 region has the nucleotide sequence shown by SEQ ID NO: 38 or 39.

5. The promoter according to claim 2, which has the nucleotide sequence shown by SEQ ID NO: 35.

6. The promoter according to claim 2, which has the nucleotide sequence shown by SEQ ID NO: 36.

7. A DNA comprising the promoter according to any one of claims 1 to 6.

8. A recombinant vector comprising the promoter according to any one of claims 1 to 6 or the DNA according to claim 7.

9. The recombinant vector according to claim 8, which comprises a DNA having a structural gene to be expressed under the control of the promoter according to any one of claims 1 to 6.

10. The recombinant vector according to claim 9, referred to as pNP3GHNO12, in which the structural gene is the human gorwth hormone gene.

11. A transformant transformed with the recombinant vector according to claim 9.

12. The transformant *Escherichia coli* MM294/pNP3GHNO12 (FERM BP-7611), which is transformed with the the recombinant vector according to claim 10.

13. A method of producing a protein or a salt thereof, which comprises culturing the transformant according to claim 11 to produce the protein encoded by the structural gene.

14. A method of producing the human growth hormone or a salt thereof, which comprises culturing the transfomlant according to claim 12 to produce the human growth hormone.

15. The recombinant vector according to claim 9, in which the structural gene is a reporter gene.

16. The recombinant vector according to claim 15, in which the reporter gene is kanamycin resistance gene or GFP gene.

17. A transformant transformed with the recombinant vector according to claim 15.

18. A transformant transformed with the recombinant vector according to claim 16.

19. A DNA comprising the same or substantially the same nucleotide sequence as that shown by SEQ ID NO: 34.

20. The DNA according to claim 20, which comprises the same or substantially the same nucleotide sequence as that shown by SEQ ID NO: 34 between any type of -35 region and any type of -10 region.

21. Use of the DNA according to claim 19 or 20 for the production of a promoter.

22. Use of the promoter according to any one of claims 1 to 6 for the production of a protein encoded by a structural gene.
